# EUROPEAN PATENT APPLICATION

(11) **EP 4 825 361 A1**
(43) Date of publication of application: **21.10.2026**
(21) Application number: 24903625.2
(22) Date of filing: 06.12.2024
(51) Int. Cl.: A61K 8/02, A61K 8/19, A61K 8/36, A61Q 5/00, C11D 1/00, C11D 3/10, C11D 3/12, C11D 3/20, C11D 17/06

(54) **CARBONIC ACID FOAMING COMPOSITION AND DETERGENT COMPOSITION**

(30) Priority: 14.12.2023 JP 2023211330; 14.12.2023 JP 2023211333
(71) Applicant: Kao Corporation, Tokyo 103-8210 (JP)
(72) Inventor: IKEDA Manato, Tokyo 103-8210 (JP); HAYASHIDA Yumi, Tokyo 103-8210 (JP); TAKEDA Saki, Tokyo 103-8210 (JP)
(74) Representative: Hoffmann Eitle
(86) International application number: PCT/JP2024/043205
(87) International publication number: WO 2025/126970

(57) **Abstract**

Provided is a carbonic acid foaming composition containing a carbonate and an organic acid, wherein the carbonic acid foaming composition contains particles (A) containing the carbonate and particles (B) containing the organic acid, the particles (A) are particles containing no organic acid, and the particles (B) are particles containing no carbonate, a median diameter of carbonate particles (a) as a starting material of the particles (A) is 170 µm or smaller, and a median diameter of organic acid particles (b) as a starting material of the particles (B) is 500 µm or smaller, a median diameter of particles of at least either the particles (A) or the particles (B) is 80 µm or larger, the median diameter of the particles (A) is equal to or larger than the median diameter of the carbonate particles (a), and the median diameter of the particles (B) is equal to or larger than the median diameter of the organic acid particles (b).

## Description

### Field of the Invention

The present invention relates to a carbonic acid foaming composition and a cleansing composition.

### Background of the Invention

Foam-type shampoos, face washes, and body soaps have been receiving attention in recent years from the viewpoint of being easily available.

In these circumstances, a foamable cosmetic composition which generates fine foam of carbonic acid gas by using a property that the addition of a small amount of water to a mixture of a carbonate and an organic acid generates carbonic acid gas, has been reported (JP-A-1989-290615: Patent Literature 1). The patent literature discloses that this foamable cosmetic composition can be used as a facial soap, a shaving foam, a shampoo, or a body soap. However, the foamable cosmetic composition described in Patent Literature 1 generates carbonic acid gas during preservation if water is present, even in a very small amount, in the mixture of the carbonate and the organic acid. Furthermore, since water is generated as a by-product through reaction, a chain reaction occurs, disadvantageously resulting in the swelling of a packaging material or reduced foamability upon application.

To solve this problem, a technique for improving preservation stability has been reported. For example, JP-A-2009-62319 (Patent Literature 2) discloses foamable granules containing an oil component and having a particle size of from 150 µm to 1 500 µm, whereby preservation stability is improved.

JP-A-2009-155213 (Patent Literature 3) discloses a bath agent composition containing the following components (A) to (C): (A) 25 to 55% by mass of a carbonic acid alkali metal salt containing 50% or more of particles having a particle size of 180 µm or larger, (B) 40 to 70% by mass of an organic acid containing 50% or more of particles having a particle size of 180 µm or larger, and (C) 0.01 to 10% by mass of a poorly water-soluble metal oxide, whereby preservation stability is favorable.

### Summary of the Invention

The present invention relates to the following [1] to [4].
[1] A carbonic acid foaming composition containing a carbonate and an organic acid, wherein
   the carbonic acid foaming composition contains particles (A) containing the carbonate and particles (B) containing the organic acid,
   the particles (A) are particles containing no organic acid, and the particles (B) are particles containing no carbonate,
   a median diameter of carbonate particles (a) as a starting material of the particles (A) is 170 µm or smaller, and a median diameter of organic acid particles (b) as a starting material of the particles (B) is 500 µm or smaller,
   a median diameter of particles of at least either the particles (A) or the particles (B) is 80 µm or larger,
   the median diameter of the particles (A) is equal to or larger than the median diameter of the carbonate particles (a), and the median diameter of the particles (B) is equal to or larger than the median diameter of the organic acid particles (b).
[2] A method for producing the carbonic acid foaming composition according to [1], containing a step of,
   by using carbonate particles (a) having a median diameter of 170 µm or smaller and organic acid particles (b) having a median diameter of 500 µm or smaller as starting materials, granulating at least either the carbonate particles (a) or the organic acid particles (b) to prepare granulated particles having a median diameter of 80 µm or larger.
[3] A cleansing composition containing a carbonate, an organic acid, and a surfactant, wherein
   the cleansing composition contains particles (A) containing the carbonate and particles (B) containing the organic acid,
   the particles (A) are particles containing no organic acid, and the particles (B) are particles containing no carbonate,
   a median diameter of carbonate particles (a) as a starting material of the particles (A), and a median diameter of organic acid particles (b) as a starting material of the particles (B) are 170 µm or smaller,
   a median diameter of particles of at least either the particles (A) or the particles (B) is 80 µm or larger,
   the median diameter of the particles (A) is equal to or larger than the median diameter of the carbonate particles (a), and the median diameter of the particles (B) is equal to or larger than the median diameter of the organic acid particles (b).
[4] A method for producing the cleansing composition according to [3], containing a step of,
   by using carbonate particles (a) having a median diameter of 170 µm or smaller and organic acid particles (b) having a median diameter of 170 µm or smaller as starting materials, granulating at least either the carbonate particles (a) or the organic acid particles (b) to prepare granulated particles having a median diameter of 80 µm or larger.

### Detailed Description of the Invention

The technique described in JP-A-2009-62319 (Patent Literature 2) employs a compression granulation method and therefore reduces the instant solubility of granules. Hence, in the case of applying this technique to a carbonic acid foaming composition to be used in making lather with a shampoo or the like by hand, a component left undissolved has been found to cause roughness, which deteriorates texture upon application and impairs feeling in use. The technique of JP-A-2009-155213 (Patent Literature 3) relates to a bath agent. When a bath agent composition is dissolved on the surface of hot bath water, carbonic acid gas dissipates without being sufficiently dissolved in the hot bath water. Therefore, in order to dissolve the bath agent composition in hot bath water, the bath agent composition needs to be immersed in the hot bath water so as to stay in the hot bath water for a given time. Accordingly, this technique is predicated on use of starting material particles having a large particle size to a given degree as particles constituting the bath agent composition. By contrast, a carbonic acid foaming composition to be used in making lather by hand requires instant solubility which permits instant dissolution in a short time in a small amount of water. Therefore, particles having a large particle size, if used in the carbonic acid foaming composition, have poor solubility, causing a roughness problem, as in the case described above. Unlike a bath agent, the carbonic acid foaming composition is required to offer delicate feel (texture) because the composition is applied to hair or the body and has direct skin contact.

Thus, the present invention relates to providing a carbonic acid foaming composition and a cleansing composition which have high preservation stability, exhibit reduced roughness in texture, and offer favorable feeling in use.

As a result of conducting studies, the present inventors found that the object can be attained by a carbonic acid foaming composition containing a carbonate and an organic acid, wherein the carbonate and the organic acid are each contained in the form of independent particles as particles (A) containing the carbonate (hereinafter, also simply referred to as "particles (A)") and particles (B) containing the organic acid (hereinafter, also simply referred to as "particles (B)"), respectively, median diameters of particles (a) of the carbonate (hereinafter, also referred to as "carbonate particles (a)") and particles (b) of the organic acid (hereinafter, also referred to as "organic acid particles (b)") as their respective starting materials are predetermined values or less, a median diameter of particles of at least either the particles (A) or the particles (B) is a predetermined value or more, and median diameters of the particles (A) and the particles (B) are equal to or larger than the median diameters of their respective starting material particles.

As a result of conducting studies, the present inventors found that the object can be attained by a cleansing composition containing a carbonate, an organic acid, and a surfactant wherein the carbonate and the organic acid are each contained in the form of independent particles as particles (A) containing the carbonate (hereinafter, also simply referred to as "particles (A)") and particles (B) containing the organic acid (hereinafter, also simply referred to as "particles (B)"), respectively, median diameters of particles (a) of the carbonate (hereinafter, also referred to as "carbonate particles (a)") and particles (b) of the organic acid (hereinafter, also referred to as "organic acid particles (b)") as their respective starting materials are predetermined values or less, a median diameter of particles of at least either the particles (A) or the particles (B) is a predetermined value or more, and median diameters of the particles (A) and the particles (B) are equal to or larger than the median diameters of their respective starting material particles.

The first embodiment of the present invention relates to the following:
a carbonic acid foaming composition containing a carbonate and an organic acid, wherein
the carbonic acid foaming composition contains particles (A) containing the carbonate and particles (B) containing the organic acid,
the particles (A) are particles containing no organic acid, and the particles (B) are particles containing no carbonate,
a median diameter of carbonate particles (a) as a starting material of the particles (A) is 170 µm or smaller, and a median diameter of organic acid particles (b) as a starting material of the particles (B) is 500 µm or smaller,
a median diameter of particles of at least either the particles (A) or the particles (B) is 80 µm or larger,
the median diameter of the particles (A) is equal to or larger than the median diameter of the carbonate particles (a), and the median diameter of the particles (B) is equal to or larger than the median diameter of the organic acid particles (b).

The second embodiment of the present invention relates to the following:
a cleansing composition containing a carbonate, an organic acid, and a surfactant, wherein
the cleansing composition contains particles (A) containing the carbonate and particles (B) containing the organic acid,
the particles (A) are particles containing no organic acid, and the particles (B) are particles containing no carbonate,
a median diameter of carbonate particles (a) as a starting material of the particles (A), and a median diameter of organic acid particles (b) as a starting material of the particles (B) are 170 µm or smaller,
a median diameter of particles of at least either the particles (A) or the particles (B) is 80 µm or larger,
the median diameter of the particles (A) is equal to or larger than the median diameter of the carbonate particles (a), and the median diameter of the particles (B) is equal to or larger than the median diameter of the organic acid particles (b).

The present invention can provide a carbonic acid foaming composition and a cleansing composition which have high preservation stability, exhibit reduced roughness in texture, and offer favorable feeling in use.

### [First embodiment: carbonic acid foaming composition]

The carbonic acid foaming composition according to the first embodiment of the present invention is a carbonic acid foaming composition containing a carbonate and an organic acid, wherein
the carbonic acid foaming composition contains particles (A) containing the carbonate and particles (B) containing the organic acid,
the particles (A) are particles containing no organic acid, and the particles (B) are particles containing no carbonate,
a median diameter of carbonate particles (a) as a starting material of the particles (A) is 170 µm or smaller, and a median diameter of organic acid particles (b) as a starting material of the particles (B) is 500 µm or smaller,
a median diameter of particles of at least either the particles (A) or the particles (B) is 80 µm or larger,
the median diameter of the particles (A) is equal to or larger than the median diameter of the carbonate particles (a), and the median diameter of the particles (B) is equal to or larger than the median diameter of the organic acid particles (b).

The carbonic acid foaming composition according to the first embodiment of the present invention is a powder or granular composition and is used for, for example, a surfactant-containing composition such as a shampoo or a conditioning agent to form the surfactant-containing composition into a dosage form of foam. Thus, the carbonic acid foaming composition according to the first embodiment of the present invention is applied in combination with a surfactant-containing composition to the surface of the human body including the skin and hair. Hence, the carbonic acid foaming composition is required to offer favorable feeling in use without roughness in texture in making lather, and needs to be instantly dissolved in a short time. Thus, particles constituting the carbonic acid foaming composition desirably have a small particle size. However, if the carbonic acid foaming composition has a small particle size, a moisture absorbent added thereto has been found to be insufficiently effective, be unable to suppress the generation of carbonic acid gas in a packaging material, and render preservation stability poor. As a result of conducting further studies, the present inventors also found that in order to suppress the generation of carbonic acid gas during preservation, the carbonate or the organic acid needs to have a large particle size equal to or larger than a specific value. As a result of conducting extensive studies to solve these conflicting demands, the present inventors completed the present invention by finding that both the instant solubility and the preservation stability can be achieved by setting the sizes of starting material particles and the sizes of particles constituting the carbonic acid foaming composition to be produced to specific ranges, respectively. Specifically, the carbonic acid foaming composition according to the first embodiment of the present invention can enhance preservation stability and in addition, can reduce roughness in texture upon application and produce favorable feeling in use, by setting median diameters of the carbonate particles (a) and the organic acid particles (b) to predetermined values or less, setting a median diameter of particles of at least either the particles (A) containing the carbonate or the particles (B) containing the organic acid to a predetermined value or more, setting a median diameter of the particles (A) to be equal to or larger than the median diameter of the starting material carbonate particles (a), and setting a median diameter of the particles (B) to be equal to or larger than the median diameter of the starting material organic acid particles (b).

Furthermore, the preservation stability and the foamability can be more improved by the carbonate and the organic acid each contained in the form of independent particles as the particles (A) containing the carbonate and the particles (B) containing the organic acid, respectively.

The carbonic acid foaming composition according to the first embodiment of the present invention may contain a surfactant so long as the effect of the surfactant-containing composition to be used in combination therewith is not impaired. The content of the surfactant may be less than 10% by mass, may be 7% by mass or less, or may be 5% by mass or less, and may be 1% by mass or more, may be 2% by mass or more, or may be 3% by mass or more.

The surfactant-containing composition will be described in detail in the section about a method for using the powder or granular carbonic acid composition.

The surfactant to be used in the carbonic acid foaming composition according to the first embodiment of the present invention can be the same as one to be used in the cleansing composition according to the second embodiment of the present invention mentioned later.

### <Carbonate and particles (A) containing carbonate>

The carbonic acid foaming composition according to the first embodiment of the present invention contains a carbonate. The carbonate is contained in the form of particles (A) containing the carbonate in the carbonic acid foaming composition according to the first embodiment of the present invention.

The particles (A) containing the carbonate to be used in the carbonic acid foaming composition according to the first embodiment of the present invention may be used as the starting material carbonate particles (a) as they are, may be used as granulated particles obtained by granulating the carbonate particles (a), or may be used as a mixture thereof.

The particles (A) contain no organic acid. In this context, the phrase "contain no" means being substantially free thereof, and the amount of the organic acid in the particles (A) is preferably less than 1% by mass, more preferably 0% by mass.

Examples of the carbonate used in the present invention include: carbonic acid dialkali metal salts such as sodium carbonate and potassium carbonate; and carbonic acid hydrogen alkali metal salts such as sodium bicarbonate and potassium bicarbonate. One or more of these carbonates can be used.

Among those described above, the carbonate preferably contains one or more members selected from the group consisting of sodium carbonate (Na₂CO₃), and sodium bicarbonate (NaHCO₃), and more preferably contains sodium bicarbonate, from the viewpoint of improvement in foamability. The content of the one or more members selected from the group consisting of sodium carbonate and sodium bicarbonate in the carbonate is preferably 80% by mass or more, more preferably 90% by mass or more, and preferably 100% by mass or less, more preferably 100% by mass, from the viewpoint of improvement in foamability.

The content of the carbonate in the carbonic acid foaming composition is preferably 15% by mass or more, more preferably 25% by mass or more, further more preferably 30% by mass or more, even more preferably 35% by mass or more, from the viewpoint of improvement in foamability. The content is preferably 65% by mass or less, more preferably 60% by mass or less, further more preferably 55% by mass or less, from the viewpoint of improvement in long-lasting foam. The content of the carbonate in the carbonic acid foaming composition is preferably 15% by mass or more and 65% by mass or less, more preferably 25% by mass or more and 60% by mass or less, further more preferably 30% by mass or more and 55% by mass or less, even more preferably 35% by mass or more and 55% by mass or less.

### <Organic acid and particles (B) containing organic acid>

The carbonic acid foaming composition according to the first embodiment of the present invention contains an organic acid. The organic acid is contained in the form of particles (B) containing the organic acid in the carbonic acid foaming composition according to the first embodiment of the present invention.

The particles (B) containing the organic acid to be used in the carbonic acid foaming composition according to the first embodiment of the present invention may be used as the starting material organic acid particles (b) as they are, may be used as granulated particles obtained by granulating the organic acid particles (b), or may be used as a mixture thereof.

The particles (B) contain no carbonate. In this context, the phrase "contain no" means being substantially free thereof, and the amount of the carbonate in the particles (B) is preferably less than 1% by mass, more preferably 0% by mass.

Examples of the organic acid used in the present invention include citric acid, tartaric acid, malic acid, malonic acid, pyridonecarboxylic acid, succinic acid, fumaric acid, adipic acid, glutaric acid, and ascorbic acid. One or more of these organic acids can be used. Among those described above, the organic acid preferably contains one or more members selected from the group consisting of citric acid, succinic acid, tartaric acid, and ascorbic acid, and more preferably contains citric acid, from the viewpoint of foamability, and solubility in water. The content of the one or more members selected from the group consisting of citric acid, succinic acid, tartaric acid, and ascorbic acid in the organic acid is preferably 80% by mass or more, more preferably 90% by mass or more, and preferably 100% by mass or less, more preferably 100% by mass, from the viewpoint of improvement in foamability.

The content of the organic acid in the carbonic acid foaming composition is preferably 5% by mass or more, more preferably 10% by mass or more, further more preferably 15% by mass or more, even more preferably 20% by mass or more, from the viewpoint of improvement in foamability. The content is preferably 60% by mass or less, more preferably 50% by mass or less, further more preferably 45% by mass or less, even more preferably 40% by mass or less, even more preferably 35% by mass or less, from the viewpoint of improvement in long-lasting foam. The content of the organic acid in the carbonic acid foaming composition is preferably 5% by mass or more and 60% by mass or less, more preferably 10% by mass or more and 50% by mass or less, further more preferably 10% by mass or more and 45% by mass or less, even more preferably 15% by mass or more and 40% by mass or less, even more preferably 20% by mass or more and 35% by mass or less.

In the carbonic acid foaming composition, the mass ratio of the organic acid to the carbonate [(organic acid)/carbonate] is preferably 0.05 or more, more preferably 0.1 or more, further more preferably 0.2 or more, and preferably 10 or less, more preferably 5.0 or less, further more preferably 1.0 or less, from the viewpoint of foamability. The mass ratio of the organic acid to the carbonate [(organic acid)/carbonate] is preferably 0.05 or more and 10 or less, more preferably 0.1 or more and 5.0 or less, further more preferably 0.2 or more and 1.0 or less.

The total amount of the carbonate and the organic acid in the carbonic acid foaming composition is preferably 50% by mass or more, more preferably 60% by mass or more, further more preferably 65% by mass or more, from the viewpoint of foamability, and preferably 90% by mass or less, more preferably 85% by mass or less, further more preferably 80% by mass or less, from the viewpoint of improvement in long-lasting foam. The total amount of the carbonate and the organic acid in the carbonic acid foaming composition is preferably 50% by mass or more and 90% by mass or less, more preferably 60% by mass or more and 85% by mass or less, further more preferably 65% by mass or more and 80% by mass or less.

### <Moisture absorbent>

The carbonic acid foaming composition according to the first embodiment of the present invention preferably further contains a moisture absorbent.

Examples of the moisture absorbent used in the present invention include alkaline earth metal oxides such as magnesium oxide, calcium oxide, and zinc oxide. Among those described above, magnesium oxide is preferred from the viewpoint of preservation stability and foamability.

The content of the moisture absorbent in the carbonic acid foaming composition is preferably 0.01% by mass or more, more preferably 0.1% by mass or more, further more preferably 1% by mass or more, even more preferably 2% by mass or more, even more preferably 4% by mass or more, from the viewpoint of improvement in preservation stability. The content is preferably 15% by mass or less, more preferably 12% by mass or less, further more preferably 10% by mass or less, from the viewpoint of improvement in foamability. The content of the moisture absorbent in the carbonic acid foaming composition is preferably 0.01% by mass or more and 15% by mass or less, more preferably 0.1% by mass or more and 12% by mass or less, further more preferably 1% by mass or more and 12% by mass or less, even more preferably 2% by mass or more and 10% by mass or less, even more preferably 4% by mass or more and 10% by mass or less.

### <Other components>

The carbonic acid foaming composition according to the first embodiment of the present invention may contain components which are generally used in carbonic acid foaming compositions, as other components so long as the object of the present invention is not impaired. Examples of such other components include excipients, water-soluble polymers, thickeners, natural dyes, moisturizers, anti-inflammatory agents, germicides, antiperspirants, antioxidants, fragrances, and mixtures thereof.

The carbonic acid foaming composition according to the first embodiment of the present invention is preferably substantially free of an oil component such as a fragrance from the viewpoint of foamability, and the content of the oil component is preferably less than 2% by mass, more preferably 1.5% by mass or less, further more preferably less than 1.0% by mass, even more preferably 0.5% by mass or less, even more preferably 0.1% by mass or less, even more preferably 0% by mass, in the carbonic acid foaming composition.

Examples of the excipient used in the present invention include: inorganic powders such as silicic acid, silicic anhydride (silica), magnesium silicate, calcium silicate, aluminum silicate, barium silicate, strontium silicate, diatomaceous earth, talc, sericite, mica, kaolin, montmorillonite, clay, bentonite, vermiculite, titanium oxide-coated mica (mica titanium), bismuth oxychloride, boron nitride, zirconium oxide, titanium oxide, low-order titanium oxide, tungstic acid metal salts, hydroxyapatite, zeolite, ceramic powders, chlorohydroxyaluminum, aluminum chloride, aluminum sulfate, basic aluminum bromide, basic aluminum iodide, chlorohydroxyaluminum zirconium, zinc sulfate, basic aluminum zinc lactate, aluminum oxide, calcium sulfate, barium sulfate, magnesium sulfate, colcothar, black iron oxide, yellow iron oxide, ultramarine, iron blue, chromium oxide, chromium hydroxide, calamine, and carbon black; monosaccharides such as glucose, fructose, galactose, and mannose; disaccharides such as lactose, trehalose, and maltose; starches such as corn starch and potato starch; and sugar alcohols such as mannitol, maltitol, xylitol, and erythritol. One or more of these excipients can be used.

The content of the excipient in the carbonic acid foaming composition is preferably 5% by mass or more, more preferably 10% by mass or more, and preferably 50% by mass or less, more preferably 40% by mass or less, further more preferably 30% by mass or less, from the viewpoint of solubility, granulability, foamability, and preservation stability. The content of the excipient in the carbonic acid foaming composition is, preferably 5% by mass or more and 50% by mass or less, more preferably 10% by mass or more and 40% by mass or less, further more preferably 10% by mass or more and 30% by mass or less.

The water-soluble polymer used in the present invention is preferably derived from a polysaccharide from the viewpoint of improving feeling in use. Such a water-soluble polymer can be used as a feel modifier and a binder for granulation mentioned later.

Examples of the water-soluble polymer used in the present invention include: semisynthetic water-soluble polymers such as hydroxyethylcellulose, hydroxymethylcellulose, carboxymethylcellulose, cationized hydroxyethylcellulose, cationized carboxymethylcellulose, cationized xanthan gum, cationized carrageenan, and cationized guar gum; and natural water-soluble polymers such as xanthan gum, carrageenan, and guar gum. One or more of these water-soluble polymers can be used. Among those described above, the feel modifier is preferably a water-soluble cationized polysaccharide, more preferably one or more members selected from the group consisting of cationized hydroxyethylcellulose, cationized carboxymethylcellulose, cationized xanthan gum, cationized carrageenan, and cationized guar gum, further more preferably one or more members selected from the group consisting of cationized hydroxyethylcellulose, cationized carboxymethylcellulose, and cationized guar gum, from the viewpoint of stabilizing foam generated upon contact with a surfactant-containing composition and from the viewpoint of improving feeling in use for use by addition to a hair cosmetic.

The content of the water-soluble polymer in the carbonic acid foaming composition is preferably 0.1% by mass or more, more preferably 1.0% by mass or more, further more preferably 2.5% by mass or more, and preferably 30% by mass or less, more preferably 20% by mass or less, further more preferably 10% by mass or less, even more preferably 5.0% by mass or less, from the viewpoint of stabilizing foam generated by addition to a surfactant-containing composition and from the viewpoint of improving feeling in use for use by addition to a hair cosmetic. The content of the water-soluble polymer in the carbonic acid foaming composition is preferably 0.1% by mass or more and 30% by mass or less, more preferably 1.0% by mass or more and 20% by mass or less, further more preferably 1.0% by mass or more and 10% by mass or less, even more preferably 2.5% by mass or more and 5.0% by mass or less.

The content of the feel modifier in the carbonic acid foaming composition is preferably 0.01% by mass or more, more preferably 0.3% by mass or more, further more preferably 0.8% by mass or more, even more preferably 1.3% by mass or more, even more preferably 2.3% by mass or more, and preferably 29.8% by mass or less, more preferably 25.3% by mass or less, further more preferably 19.8% by mass or less, even more preferably 9.8% by mass or less, even more preferably 4.8% by mass or less, from the viewpoint of stabilizing foam generated upon contact with a surfactant-containing composition and from the viewpoint of improving feeling in use when the carbonic acid foaming composition is added to a surfactant-containing composition such as a hair cosmetic and used. The content of the feel modifier in the carbonic acid foaming composition is preferably 0.01% by mass or more and 29.8% by mass or less, more preferably 0.3% by mass or more and 25.3% by mass or less, further more preferably 0.8% by mass or more and 19.8% by mass or less, even more preferably 1.3% by mass or more and 9.8% by mass or less, even more preferably 2.3% by mass or more and 4.8% by mass or less.

### <Particle sizes of carbonate particles (a), organic acid particles (b), particles (A), and particles (B)>

The median diameter (D50) according to the present invention means a particle size at which a cumulative volume frequency calculated from volume fractions is 50% when calculated from smaller toward larger particle sizes. Specifically, the median diameter can be measured by a method described in Examples.

The median diameter of the carbonate particles (a) refers to the median diameter of particles of the carbonate as a starting material of the particles (A) containing the carbonate and is preferably 10 µm or larger, more preferably 15 µm or larger, further more preferably 20 µm or larger, from the viewpoint of improving productivity and preservation stability. The median diameter is 170 µm or smaller, preferably 150 µm or smaller, more preferably 120 µm or smaller, further more preferably 110 µm or smaller, even more preferably 100 µm or smaller, even more preferably 95 µm or smaller, even more preferably 90 µm or smaller, from the viewpoint of attaining favorable foamability and texture. The median diameter of the carbonate particles (a) is 170 µm or smaller, preferably 10 µm or larger and 170 µm or smaller, more preferably 10 µm or larger and 150 µm or smaller, further more preferably 15 µm or larger and 120 µm or smaller, even more preferably 20 µm or larger and 110 µm or smaller, even more preferably 20 µm or larger and 100 µm or smaller, even more preferably 20 µm or larger and 95 µm or smaller, even more preferably 20 µm or larger and 90 µm or smaller.

In the case of granulating the carbonate particles (a), the median diameter of the carbonate (carbonate particles (a)) as a starting material is further more preferably in the range described above as well as even more preferably 100 µm or smaller, even more preferably 90 µm or smaller, even more preferably 70 µm or smaller, even more preferably 60 µm or smaller, from the same viewpoint as described above as well as the viewpoint of more improving foamability and texture, and is even more preferably 10 µm or larger and 100 µm or smaller, even more preferably 10 µm or larger and 90 µm or smaller, even more preferably 10 µm or larger and 70 µm or smaller, even more preferably 10 µm or larger and 60 µm or smaller.

In the case of using the carbonate particles (a) without being granulated, the median diameter of the carbonate particles (a) is further more preferably in the range described above as well as even more preferably 80 µm or larger, even more preferably 90 µm or larger, from the same viewpoint as described above as well as the viewpoint of the balance between preservation stability and texture, and is even more preferably 80 µm or larger and 170 µm or smaller, even more preferably 80 µm or larger and 150 µm or smaller, even more preferably 90 µm or larger and 120 µm or smaller, even more preferably 90 µm or larger and 110 µm or smaller.

The median diameter of the organic acid particles (b) refers to the median diameter of particles of the organic acid as a starting material of the particles (B) containing the organic acid and is preferably 10 µm or larger, more preferably 15 µm or larger, further more preferably 20 µm or larger, from the viewpoint of improving productivity and preservation stability. The median diameter is 500 µm or smaller, preferably 350 µm or smaller, more preferably 170 µm or smaller, further more preferably 150 µm or smaller, even more preferably 120 µm or smaller, even more preferably 110 µm or smaller, even more preferably 100 µm or smaller, even more preferably 95 µm or smaller, even more preferably 90 µm or smaller, from the viewpoint of attaining favorable foamability and texture. The median diameter of the organic acid particles (b) is 500 µm or smaller, preferably 10 µm or larger and 350 µm or smaller, more preferably 10 µm or larger and 170 µm or smaller, further more preferably 10 µm or larger and 150 µm or smaller, even more preferably 15 µm or larger and 120 µm or smaller, even more preferably 20 µm or larger and 110 µm or smaller, even more preferably 20 µm or larger and 100 µm or smaller, even more preferably 20 µm or larger and 95 µm or smaller, even more preferably 20 µm or larger and 90 µm or smaller.

In the case of granulating the organic acid particles (b), the median diameter of the organic acid (organic acid particles (b)) as a starting material is further more preferably in the range described above as well as even more preferably 70 µm or smaller, even more preferably 60 µm or smaller, from the same viewpoint as described above as well as the viewpoint of more improving foamability and texture, and is even more preferably 10 µm or larger and 110 µm or smaller.

In the case of using the organic acid particles (b) without being granulated, the median diameter of the organic acid particles (b) is further more preferably in the range described above as well as even more preferably 80 µm or larger, even more preferably 90 µm or larger, from the same viewpoint as described above as well as the viewpoint of the balance between preservation stability and texture, and is even more preferably 80 µm or larger and 350 µm or smaller, even more preferably 80 µm or larger and 170 µm or smaller, even more preferably 90 µm or larger and 150 µm or smaller, even more preferably 90 µm or larger and 110 µm or smaller.

For the particle sizes of the carbonate particles (a) and the organic acid particles (b), the value of the coefficient of variation, CV, represented by the following expression (1), which is calculated as the value of standard deviation σ of a particle size relative to a median diameter D is preferably 95% or less, more preferably 90% or less, further more preferably 85% or less, from the viewpoint of attaining high preservation stability, reduced roughness in texture, and favorable feeling in use. 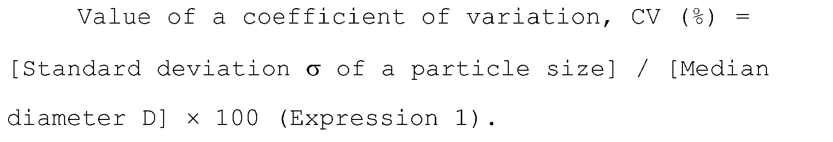 Value of a coefficient of variation, CV (%) = [Standard deviation σ of a particle size] / [Median diameter D] × 100 (Expression 1)

The median diameter of an additional starting material component other than the carbonate and the organic acid is preferably equal to or smaller than the median diameters of the carbonate particles (a) and the organic acid particles (b), for use, and is usually 170 µm or smaller, for example, 5 µm or larger and 150 µm or smaller.

In the carbonic acid foaming composition according to the first embodiment of the present invention, the median diameter of the particles of at least either the particles (A) or the particles (B) is 80 µm or larger, preferably 90 µm or larger, more preferably 100 µm or larger, further more preferably 110 µm or larger, even more preferably 120 µm or larger. When the median diameter of the particles of at least either the particles (A) or the particles (B) falls within this range, preservation stability is excellent. Both the median diameters of the particles (A) and the particles (B) are preferably 80 µm or larger, more preferably 90 µm or larger, further more preferably 100 µm or larger, from the viewpoint of further improvement in preservation stability. The median diameter of only either the particles (A) or the particles (B) is preferably 80 µm or larger, more preferably 90 µm or larger, further more preferably 100 µm or larger, from the viewpoint of improving productivity.

The median diameters of the particles (A) and the particles (B) are preferably 500 µm or smaller, more preferably 450 µm or smaller, further more preferably 400 µm or smaller, even more preferably 350 µm or smaller, from the viewpoint of productivity.

When starting material particles are used as they are as the particles (A), i.e., when the particles (A) are the carbonate particles (a), a preferred range of the median diameter of the particles (A) is the same as the suitable range of the median diameter of the carbonate particles (a) which are used without being granulated as mentioned above.

When starting material particles are used as they are as the particles (B), i.e., when the particles (B) are the organic acid particles (b), a preferred range of the median diameter of the particles (B) is the same as the suitable range of the median diameter of the organic acid particles (b) which are used without being granulated as mentioned above.

When the particles (A) have a median diameter of 80 µm or larger, the median diameter of the particles (A) is preferably 90 µm or larger, more preferably 100 µm or larger, further more preferably 110 µm or larger, even more preferably 120 µm or larger, from the viewpoint of preservation stability. The median diameter is preferably 500 µm or smaller, more preferably 400 µm or smaller, further more preferably 350 µm or smaller, even more preferably 300 µm or smaller, from the viewpoint of productivity. The median diameter of the particles (A) is preferably 80 µm or larger and 500 µm or smaller, more preferably 90 µm or larger and 400 µm or smaller, further more preferably 100 µm or larger and 350 µm or smaller, even more preferably 110 µm or larger and 350 µm or smaller, even more preferably 110 µm or larger and 300 µm or smaller, even more preferably 120 µm or larger and 300 µm or smaller. Particularly, when the particles (A) are granulated particles, the median diameter is further more preferably 110 µm or larger, even more preferably 120 µm or larger, from the same viewpoint as described above and from the viewpoint of more improving preservation stability, and is even more preferably 110 µm or larger and 350 µm or smaller, even more preferably 110 µm or larger and 300 µm or smaller, and even more preferably 120 µm or larger and 300 µm or smaller.

When the particles (B) have a median diameter of 80 µm or larger, the median diameter of the particles (B) is preferably 90 µm or larger, more preferably 100 µm or larger, further more preferably 110 µm or larger, even more preferably 120 µm or larger, from the viewpoint of preservation stability. The median diameter is preferably 500 µm or smaller, more preferably 400 µm or smaller, further more preferably 350 µm or smaller, even more preferably 300 µm or smaller from the viewpoint of productivity. The median diameter of the particles (B) is preferably 80 µm or larger and 500 µm or smaller, more preferably 90 µm or larger and 400 µm or smaller, further more preferably 100 µm or larger and 350 µm or smaller, even more preferably 110 µm or larger and 350 µm or smaller, even more preferably 110 µm or larger and 300 µm or smaller, even more preferably 120 µm or larger and 300 µm or smaller. Particularly, when the particles (B) are granulated particles, the median diameter is further more preferably 110 µm or larger, even more preferably 120 µm or larger, from the same viewpoint as described above and from the viewpoint of more improving preservation stability, and is even more preferably 110 µm or larger and 350 µm or smaller, even more preferably 110 µm or larger and 300 µm or smaller, and even more preferably 120 µm or larger and 300 µm or smaller.

In the carbonic acid foaming composition according to the first embodiment of the present invention, the median diameter of the particles (A) is equal to or larger than the median diameter of the carbonate particles (a), and the median diameter of the particles (B) is equal to or larger than the median diameter of the organic acid particles (b).

When the median diameter of the carbonate particles (a) falls within the range of the median diameter of the particles (A), the carbonate particles (a) can be used as they are as the particles (A). Likewise, when the median diameter of the organic acid particles (b) falls within the range of the median diameter of the particles (B), the organic acid particles (b) can be used as they are as the particles (B).

Alternatively, in the case of setting the median diameter of the particles (A) to be larger than that of the carbonate particles (a), the size can be adjusted by, for example, granulation mentioned later. Likewise, in the case of setting the median diameter of the particles (B) to be larger than that of the organic acid particles (b), the size can be adjusted by, for example, granulation mentioned later.

### <Granulated particles>

In the first embodiment of the present invention, the median diameter of the particles (A) containing the carbonate is preferably set to be larger than that of the carbonate particles (a) by granulating the starting material carbonate particles (a), from the viewpoint of attaining high preservation stability, reduced roughness in texture, and favorable feeling in use.

The median diameter of the particles (B) containing the organic acid is preferably set to be larger than that of the organic acid particles (b) by granulating the starting material organic acid particles (b), from the viewpoint of attaining high preservation stability, reduced roughness in texture, and favorable feeling in use.

When the particles (A) containing the carbonate are granulated particles, the particles (A) are preferably obtained by granulating the carbonate particles (a) together with one or more members selected from the group consisting of components other than the organic acid, for example, an excipient, a water-soluble polymer (a binder and a feel modifier), and a moisture absorbent, particularly preferably all the components other than the organic acid, from the viewpoint of reducing the amount of a fine powder, though they may be granulated particles obtained by granulating the carbonate particles (a) alone.

When the particles (B) containing the organic acid are granulated particles, the particles (B) are preferably obtained by granulating the organic acid particles (b) together with one or more members selected from the group consisting of components other than the carbonate, for example, an excipient, a water-soluble polymer (a binder and a feel modifier), and a moisture absorbent, particularly preferably all the components other than the carbonate, from the viewpoint of reducing the amount of a fine powder, though they may be granulated particles obtained by granulating the organic acid particles (b) alone.

The particles (A) containing the carbonate or the particles (B) containing the organic acid are preferably granulated particles further containing the moisture absorbent. The granulation together with the moisture absorbent can reduce the amount of a fine powder derived from the moisture absorbent, and can prevent choking on a fine powder flying upon application of the carbonic acid foaming composition.

The particles of at least either the particles (A) containing the carbonate or the particles (B) containing the organic acid preferably contain granulated particles obtained by granulating starting material particles, from the viewpoint of improving preservation stability and attaining favorable feeling in use.

Particles having a median diameter of 80 µm or larger in the carbonic acid foaming composition preferably contain granulated particles obtained by granulating starting material particles, from the viewpoint of improving preservation stability and attaining favorable feeling in use. In this context, the "starting material particles" refer to at least either the carbonate particles (a) or the organic acid particles (b), and the "granulated particles obtained by granulating starting material particles" refer to the particles (A) when the carbonate particles (a) are granulated, refer to the particles (B) when the organic acid particles (b) are granulated, and refer to the particles (A) and the particles (B) when the carbonate particles (a) and the organic acid particles (b), respectively, are granulated.

Both the particles (A) containing the carbonate and the particles (B) containing the organic acid are preferably separately granulated from the viewpoint of improving preservation stability and attaining favorable feeling in use. Alternatively, it is preferred that only either the particles (A) or the particles (B) should be granulated particles and the other particles should be used as starting material particles, from the viewpoint of productivity, and it is preferred that the particles (B) containing the organic acid should be granulated particles and the carbonate particles (a) as starting material particles should be used as they are as the particles (A) containing the carbonate, from the viewpoint of production efficiency.

For the particles (A) and the particles (B), the particles of at least either the particles (A) or the particles (B) are preferably granulated with one or more members selected from the group consisting of components other than the carbonate particles (a) and the organic acid particles (b), for example, an excipient, a water-soluble polymer (a binder and a feel modifier), and a moisture absorbent, particularly preferably with all the components other than the carbonate particles (a) and the organic acid particles (b), from the viewpoint of reducing the amount of a fine powder. Both the particles (A) and the particles (B) are preferably granulated from the viewpoint of more improving preservation stability and from the viewpoint of improving instant solubility, and either the particles (A) or the particles (B) are preferably granulated from the viewpoint of improving productivity.

The particles (A) or the particles (B) preferably contain the excipient, or both the particles (A) and the particles (B) preferably contain the excipient. Specifically, the carbonic acid foaming composition according to the first embodiment of the present invention may contain the carbonate particles (a) and particles (B) containing the organic acid and the excipient, may contain particles (A) containing the carbonate and the excipient and the organic acid particles (b), or may contain particles (A) containing the carbonate and the excipient and particles (B) containing the organic acid and the excipient.

In the carbonic acid foaming composition according to the first embodiment of the present invention, the content of a 10 µm or smaller fine powder is preferably 3.5% by mass or less, more preferably 3.3% by mass or less, further more preferably 3.0% by mass or less, in all particles constituting the carbonic acid foaming composition from the viewpoint of improving feeling in use and, particularly, preventing choking on the fine powder. The content is preferably 0.001% by mass or more, more preferably 0.002% by mass or more, further more preferably 0.003% by mass or more, from the viewpoint of productivity. The content of the 10 µm or smaller fine powder is preferably 0.001% by mass or more and 3.5% by mass or less, more preferably 0.002% by mass or more and 3.3% by mass or less, further more preferably 0.003% by mass or more and 3.0% by mass or less, in all particles constituting the carbonic acid foaming composition.

### <Granulation method>

Any of a fluidized-bed granulation method, a stirring granulation method, a tumbling granulation method, and an extrusion granulation method can be used as a granulation method for the particles (A) or the particles (B). Among those described above, a fluidized-bed granulation method is preferred from the viewpoint of improving the solubility of the carbonic acid foaming composition in a liquid (water).

Specifically, in the case of granulating, for example, the particles (A), granulation can be carried out by charging starting materials other than the organic acid into a fluidized-bed granulator and adding a binder solution of a binder dissolved in water, ethanol, or a mixed liquid thereof to a uniformly mixed powder and granular material. Likewise, in the case of granulating the particles (B), granulation can be carried out by charging starting materials other than the carbonate into a fluidized-bed granulator and adding a binder solution of a binder dissolved in water, ethanol, or a mixed liquid thereof to a uniformly mixed powder and granular material.

The binder for use in granulation in the present invention is preferably selected from the group consisting of the water-soluble polymers described above. One or more members selected from the group consisting of hydroxyethylcellulose, hydroxymethylcellulose, carboxymethylcellulose, xanthan gum, carrageenan, and guar gum can be used from the viewpoint of the instant solubility and productivity of the carbonic acid foaming composition, and one or more members selected from the group consisting of hydroxyethylcellulose, hydroxymethylcellulose, carboxymethylcellulose, xanthan gum, and carrageenan are preferably used.

The content (solid content) of the binder in the binder solution is preferably 0.1% by mass or more, more preferably 0.3% by mass or more, further more preferably 0.5% by mass or more, and preferably 10% by mass or less, more preferably 8% by mass or less, further more preferably 5% by mass or less, from the viewpoint of productivity. The content of the binder in the binder solution is preferably from 0.1 to 10% by mass, more preferably from 0.3 to 8% by mass, further more preferably from 0.5 to 5% by mass.

The content (solid content) of the binder in the carbonic acid foaming composition is preferably 0.01% by mass or more, more preferably 0.05% by mass or more, further more preferably 0.1% by mass or more, and preferably 10% by mass or less, more preferably 5% by mass or less, further more preferably 3% by mass or less, from the viewpoint of instant solubility. The content of the binder in the carbonic acid foaming composition is preferably from 0.01 to 10% by mass, more preferably from 0.05 to 5% by mass, further more preferably from 0.1 to 3% by mass.

### <Method for producing carbonic acid foaming composition>

The method for producing the carbonic acid foaming composition according to the first embodiment of the present invention contains a step of, by using carbonate particles (a) having a median diameter of 170 µm or smaller and organic acid particles (b) having a median diameter of 500 µm or smaller as starting materials, granulating at least either the carbonate particles (a) or the organic acid particles (b) to prepare granulated particles having a median diameter of 80 µm or larger (hereinafter, also referred to as a "granulated particle preparation step").

Carbonate particles (a) having a median diameter of 170 µm or smaller and organic acid particles (b) having a median diameter of 500 µm or smaller are used as starting material particles, and at least either them is granulated so as to be 80 µm or larger, whereby preservation stability can be improved while the carbonic acid foaming composition thus produced is excellent in solubility, exhibits reduced roughness in texture, and offers favorable feeling in use.

The granulated particle preparation step can be performed by the granulation method mentioned above.

The granulation of at least either the carbonate particles (a) or the organic acid particles (b) includes the case of separately granulating both the carbonate particles (a) and the organic acid particles (b) and the case of granulating only either the carbonate particles (a) or the organic acid particles (b). Specifically, either the carbonate particles (a) or the organic acid particles (b) may be used as starting material particles without being granulated, or the carbonate particles (a) and the organic acid particles (b) may be granulated and used as granulated particles (A) of the carbonate and granulated particles (B) of the organic acid, respectively. The carbonate particles (a) and the organic acid particles (b) are preferably separately granulated from the viewpoint of more improving preservation stability and from the viewpoint of improving instant solubility. Only either the carbonate particles (a) or the organic acid particles (b) are preferably granulated from the viewpoint of improving productivity.

After the granulated particle preparation step, the granulated particles (particles (A) and particles (B)) in the case of separately granulating the carbonate particles (a) and the organic acid particles (b), or the granulated particles and ungranulated particles in the case of granulating either the carbonate particles (a) or the organic acid particles (b) can be mixed by a known means to produce a carbonic acid foaming composition.

### <Method for using carbonic acid foaming composition>

The carbonic acid foaming composition according to the first embodiment of the present invention is used by bringing the composition into contact with a surfactant-containing composition upon application to form the surfactant-containing composition into a dosage form of foam.

More specifically, the carbonic acid foaming composition according to the first embodiment of the present invention is brought into contact with a surfactant-containing composition upon application, whereby carbonic acid gas is generated through the reaction between the carbonate and the organic acid contained in the carbonic acid foaming composition by a liquid component (water) contained in the surfactant-containing composition or an optionally added liquid component (water), and the surfactant-containing composition can thereby be formed into a dosage form of foam.

The carbonic acid foaming composition according to the first embodiment of the present invention starts to foam spontaneously through the reaction with water, and the composition makes quick lather with the surfactant-containing composition and eliminates the need of a special procedure of making lather, which requires time and labor. Thus, the carbonic acid foaming composition according to the first embodiment of the present invention can be conveniently utilized.

The method for using the carbonic acid foaming composition according to the first embodiment of the present invention is also a foaming method and a washing method.

Specifically, the method also serves as a foaming method for a surfactant composition, containing bringing the carbonic acid foaming composition according to the first embodiment of the present invention into contact with the surfactant-containing composition to form the surfactant-containing composition into a dosage form of foam.

The method also serves as a method for washing the body, containing: bringing the carbonic acid foaming composition according to the first embodiment of the present invention into contact with a surfactant-containing composition to form the surfactant-containing composition into a dosage form of foam; and applying the surfactant composition in the form of foam to the surface of the human body for washing.

The method also serves as a hair washing method, containing: bringing the carbonic acid foaming composition according to the first embodiment of the present invention into contact with a surfactant-containing composition to form the surfactant-containing composition into a dosage form of foam; and applying the surfactant composition in the form of foam to hair for washing.

In the present specification, the surfactant-containing composition is not particularly limited so long as the composition contains a surfactant and is applicable to the surface of the human body including the skin and hair. Examples thereof include hair cosmetics, face washes, and body cleansers (body shampoos). Examples of the hair cosmetic include shampoos, rinses, conditioning agents, treatment agents, styling agents, hair dyes, and hair growth tonics.

Among those described above, the surfactant-containing composition is preferably a hair cosmetic, more preferably one member selected from the group consisting of a shampoo, a rinse, a conditioning agent, and a treatment agent, further more preferably a shampoo, from the viewpoint of being capable of more effectively exerting the advantageous effects of the present invention.

The surfactant-containing composition is also preferably in a liquid state from the viewpoint of more effectively exerting the advantageous effects of the present invention.

A hair cosmetic, when applied directly to hair, may impose a burden on the hair or the scalp by causing the friction between the hair and the scalp in making lather or spreading over the hair. However, a hair cosmetic used in combination with the carbonic acid foaming composition according to the first embodiment of the present invention can be applied in the form of foam, whereby the burden on hair or the scalp can be reduced. Furthermore, the carbonic acid foaming composition according to the first embodiment of the present invention is excellent in instant solubility and is therefore also excellent in feeling in use such as feel (texture) even when dissolved in a hair cosmetic. Moreover, the carbonic acid foaming composition according to the first embodiment of the present invention is excellent in versatility because the composition can be used in combination with a commercially available hair cosmetic such as a shampoo or a conditioning agent and eliminates the need of a special container or tool for a procedure of making lather. Particularly, the composition enclosed as a portion for one usage in a packaging material can be easy to carry and is therefore also available while on the go. Besides, a blood circulation-promoting effect by carbonic acid gas can also be expected.

The method for using the carbonic acid foaming composition according to the first embodiment of the present invention is more specifically performed, for example, by putting the carbonic acid foaming composition on the palm, adding the surfactant-containing composition to the carbonic acid foaming composition, thoroughly blending the surfactant-containing composition into the carbonic acid foaming composition on the palm, allowing the surfactant-containing composition to foam spontaneously, then lightly making lather, and applying the foam to a recipient such as the face, hair, or the whole body. The order of putting these compositions on the palm is not limited, and the carbonic acid foaming composition is preferably put on the palm first, followed by the addition of the surfactant-containing composition thereto, from the viewpoint of reducing scattering of the carbonic acid foaming composition and more effectively causing foaming of the surfactant-containing composition, though the surfactant-containing composition may be put on the palm, followed by the addition of the carbonic acid foaming composition to the surfactant-containing composition.

If the foaming of the surfactant-containing composition is unlikely to proceed, water may be further added thereto, if necessary. In this respect, the timing of adding water is not particularly limited, and, for example, the surfactant-containing composition may be added after the carbonic acid foaming composition is brought into contact with water, or the carbonic acid foaming composition may be added after the surfactant-containing composition is brought into contact with water. Alternatively, water may be added after the carbonic acid foaming composition is brought into contact with the surfactant-containing composition. The surfactant composition is preferably added after the carbonic acid foaming composition is brought into contact with water from the viewpoint of more effectively causing foaming of the surfactant-containing composition. However, use without the addition of water is preferred from the viewpoint of improvement in feeling in use.

When the surfactant-containing composition is in a liquid state, the amount of the liquid surfactant-containing composition to be added to the carbonic acid foaming composition is preferably 1/30 or more, more preferably 1/25 or more, further more preferably 1/20 or more, and preferably 1/0.5 or less, more preferably 1/1 or less, further more preferably 1/2 or less, in terms of the mass ratio of the carbonic acid foaming composition and the surfactant-containing composition to be added [(carbonic acid foaming composition)/(surfactant-containing composition)] from the viewpoint of improving the solubility of the carbonic acid foaming composition and also improving foamability. The mass ratio [(carbonic acid foaming composition)/(surfactant-containing composition)] is preferably 1/30 or more and 1/0.5 or less, more preferably 1/25 or more and 1/1 or less, further more preferably 1/20 or more and 1/2 or less.

The amount of the water added, if necessary, is preferably 0.1 g or more and 20 g or less, more preferably 0.5 g or more and 15 g or less, further more preferably 1 g or more and 10 g or less, per g of the carbonic acid foaming composition from the viewpoint of solubility and foamability.

The temperature of the water to be added, if necessary, to the carbonic acid foaming composition is preferably 15°C or higher and 50°C or lower, more preferably 15°C or higher and 45°C or lower, further more preferably 20°C or higher and 45°C or lower, though it is not particularly limited.

When the surfactant-containing composition is in a liquid state, the carbonic acid foaming composition according to the first embodiment of the present invention is applied to an object such as hair within preferably 5 minutes, more preferably 3 minutes, further more preferably 2 minutes, even more preferably 1.5 minutes, after contact with the liquid surfactant-containing composition (or after contact of the carbonic acid foaming composition with water when the carbonic acid foaming composition is brought into contact with water first) from the viewpoint of the retention of foam.

The carbonic acid foaming composition according to the first embodiment of the present invention can be suitably used as a foaming aid which helps a surfactant-containing composition, particularly, a hair cosmetic such as a shampoo or a conditioning agent, make lather.

The carbonic acid foaming composition according to the first embodiment of the present invention can be enclosed in a packaging material and provided. The carbonic acid foaming composition according to the first embodiment of the present invention is excellent in preservation stability in the form of a product enclosed in a packaging material because the composition, even when enclosed and preserved in the packaging material, is unlikely to cause the swelling of the packaging material ascribable to the generation of carbonic acid gas.

The shape of the packaging material is not particularly limited so long as it has a structure where the carbonic acid foaming composition can be enclosed. Examples thereof include bag and bottle shapes. Among them, a bag-shaped packaging material is preferred.

A material constituting the packaging material is not particularly limited so long as the carbonic acid foaming composition can be enclosed therein. For example, a resin film, or a laminated film of an inorganic thin film of a metal or a metal oxide laminated on a resin film can be used for a bag-shaped packaging material.

### <Hair cosmetic kit>

The hair cosmetic kit according to the first embodiment of the present invention contains a carbonic acid foaming composition and a surfactant-containing composition.

The carbonic acid foaming composition and the surfactant-containing composition for use in the hair cosmetic kit according to the first embodiment of the present invention are the same as those mentioned above. The hair cosmetic kit is used by bringing the carbonic acid foaming composition into contact with the surfactant-containing composition upon application to form the surfactant-containing composition into a dosage form of foam.

### [Second embodiment: cleansing composition]

The cleansing composition according to the second embodiment of the present invention is a cleansing composition containing a carbonate, an organic acid, and a surfactant, wherein
the cleansing composition contains particles (A) containing the carbonate and particles (B) containing the organic acid,
the particles (A) are particles containing no organic acid, and the particles (B) are particles containing no carbonate,
a median diameter of carbonate particles (a) as a starting material of the particles (A), and a median diameter of organic acid particles (b) as a starting material of the particles (B) are 170 µm or smaller,
a median diameter of particles of at least either the particles (A) or the particles (B) is 80 µm or larger,
the median diameter of the particles (A) is equal to or larger than the median diameter of the carbonate particles (a), and the median diameter of the particles (B) is equal to or larger than the median diameter of the organic acid particles (b).

The cleansing composition according to the second embodiment of the present invention can exert the washing effect of the surfactant as well as excellent washing performance by generating carbonic acid gas through the reaction of the carbonate and the organic acid when the cleansing composition is dissolved in water.

The cleansing composition according to the second embodiment of the present invention is powder or granular, typically used for washing the face, the body, or the like. Such a cleansing composition to be applied to the skin such as the face or the body is required to offer favorable feeling in use without roughness in texture in making lather, and needs to be instantly dissolved in a short time. Thus, particles constituting the cleansing composition desirably have a small particle size. However, if the cleansing composition has a small particle size, a moisture absorbent added thereto has been found to be insufficiently effective, be unable to suppress the generation of carbonic acid gas in a packaging material, and render preservation stability poor. As a result of conducting further studies, the present inventors also found that in order to suppress the generation of carbonic acid gas during preservation, the carbonate or the organic acid needs to have a large particle size equal to or larger than a specific value. As a result of conducting extensive studies to solve these conflicting demands, the present inventors found that both the instant solubility and the preservation stability can be achieved by setting the sizes of starting material particles and the sizes of particles constituting the cleansing composition to be produced to specific ranges, respectively. Specifically, the cleansing composition according to the second embodiment of the present invention can enhance preservation stability and in addition, can reduce roughness in texture and produce favorable feeling in use, by setting median diameters of the carbonate particles (a) and the organic acid particles (b) to predetermined values or less, setting a median diameter of particles of at least either the particles (A) containing the carbonate or the particles (B) containing the organic acid to a predetermined value or more, setting a median diameter of the particles (A) to be equal to or larger than the median diameter of the starting material carbonate particles (a), and setting a median diameter of the particles (B) to be equal to or larger than the median diameter of the starting material organic acid particles (b) .

Furthermore, the preservation stability and the foamability can be more improved by the carbonate and the organic acid each contained in the form of independent particles as the particles (A) containing the carbonate and the particles (B) containing the organic acid, respectively.

### <Carbonate and particles (A) containing carbonate>

The cleansing composition according to the second embodiment of the present invention contains a carbonate. The carbonate is contained in the form of particles (A) containing the carbonate in the cleansing composition of the present invention.

The particles (A) containing the carbonate for use in the cleansing composition according to the second embodiment of the present invention may be used as the starting material carbonate particles (a) as they are, may be used as granulated particles obtained by granulating the carbonate particles (a), or may be used as a mixture thereof.

The particles (A) contain no organic acid. In this context, the phrase "contain no" means being substantially free thereof, and the amount of the organic acid in the particles (A) is preferably less than 1% by mass, more preferably 0% by mass.

The carbonate to be used in the cleansing composition according to the second embodiment of the present invention is as described in the first embodiment, and its preferred range is also the same.

The content of the carbonate in the cleansing composition according to the second embodiment of the present invention is preferably 10% by mass or more, more preferably 15% by mass or more, further more preferably 20% by mass or more, even more preferably 30% by mass or more, from the viewpoint of improvement in foamability. The content is preferably 60% by mass or less, more preferably 55% by mass or less, further more preferably 50% by mass or less, from the viewpoint of improvement in long-lasting foam. The content of the carbonate in the cleansing composition is preferably 10% by mass or more and 60% by mass or less, more preferably 15% by mass or more and 55% by mass or less, further more preferably 20% by mass or more and 50% by mass or less, even more preferably 30% by mass or more and 50% by mass or less.

### <Organic acid and particles (B) containing organic acid>

The cleansing composition according to the second embodiment of the present invention contains an organic acid. The organic acid is contained in the form of particles (B) containing the organic acid in the cleansing composition according to the second embodiment of the present invention.

The particles (B) containing the organic acid to be used in the cleansing composition according to the second embodiment of the present invention may be used as the starting material organic acid particles (b) as they are, may be used as granulated particles obtained by granulating the organic acid particles (b), or may be used as a mixture thereof.

The particles (B) contain no carbonate. In this context, the phrase "contain no" means being substantially free thereof, and the amount of the carbonate in the particles (B) is preferably less than 1% by mass, more preferably 0% by mass.

The organic acid to be used in the cleansing composition according to the second embodiment of the present invention is as described in the first embodiment, and its preferred range is also the same.

The content of the organic acid in the cleansing composition according to the second embodiment of the present invention is preferably 5% by mass or more, more preferably 7% by mass or more, further more preferably 10% by mass or more, from the viewpoint of improvement in foamability. The content is preferably 60% by mass or less, more preferably 50% by mass or less, further more preferably 40% by mass or less, even more preferably 30% by mass or less, even more preferably 20% by mass or less, from the viewpoint of improvement in long-lasting foam. The content of the organic acid in the cleansing composition is preferably 5% by mass or more and 60% by mass or less, more preferably 5% by mass or more and 50% by mass or less, further more preferably 7% by mass or more and 40% by mass or less, even more preferably 7% by mass or more and 30% by mass or less, even more preferably 10% by mass or more and 20% by mass or less.

### <Surfactant>

The cleansing composition according to the second embodiment of the present invention contains a surfactant.

Examples of the surfactant to be used in the cleansing composition according to the second embodiment of the present invention include anionic surfactants, cationic surfactants, amphoteric surfactants, and nonionic surfactants. One or more of these surfactants can be used. Hereinafter, their respective specific examples will be shown.

### [Anionic surfactant]

Specific examples of the anionic surfactant include N-acylamino acid salts, N-acyl-N-methylamino acid salts, fatty acid salts, ester salts with fatty acids having 5 or more and 18 or less carbon atoms and isethionic acid, alkyl or alkenyl sulfonates having 10 or more and 18 or less carbon atoms, polyoxyalkylene alkyl ether sulfuric acid ester salts, and linear alkyl benzenesulfonates.

Examples of the counterion of an anionic group in the anionic surfactant include: alkali metal ions such as sodium ions and potassium ions; alkaline earth metal ions such as calcium ions and magnesium ions; ammonium ions; and alkanol ammonium having one to three alkanol groups having 2 or 3 carbon atoms (e.g., monoethanol ammonium, diethanol ammonium, triethanol ammonium, and triisopropanol ammonium). Sodium ions or potassium ions are preferred, and sodium ions are more preferred.

### [Cationic surfactant]

Examples of the cationic surfactant include quaternary ammonium salts such as alkyl trimethyl ammonium salts, alkoxyalkyl trimethyl ammonium salts, dialkyl dimethyl ammonium salts, alkylamide alkyl trimethyl ammonium salts, benzalkonium chloride, and alkylpyridinium salts.

Examples of the counterion of a cationic group in the cationic surfactant include alkyl sulfate ions having 1 or more and 3 or less carbon atoms, sulfate ions, phosphate ions, carboxylate ions having 1 or more and 3 or less carbon atoms (formate ions, acetate ions, and propionate ions), and halide ions such as chloride ions and bromide ions. Among them, halide ions are preferred, and chloride ions are more preferred, from the viewpoint of easy production and easy availability of a starting material.

### [Amphoteric surfactant]

Examples of the amphoteric surfactant include one or more members selected from the group consisting of alkylamine oxides having an alkyl group having 10 or more and 18 or less carbon atoms, and alkyl betaines having an alkyl group having 10 or more and 18 or less carbon atoms.

### [Nonionic surfactant]

Specific examples of the nonionic surfactant include one or more members selected from the group consisting of polyoxyethylene alkyl ethers, polyoxyethylene alkenyl ethers, polyoxyethylene sorbitan fatty acid esters, polyoxyethylene sorbitol fatty acid esters, polyoxyethylene fatty acid esters, alkyl glucosides, alkylalkanolamides, alkyl glyceryl ethers, higher fatty acid sucrose esters, polyglycerin fatty acid esters, polyoxyethylene hydrogenated castor oil, and alkyl saccharides.

Among those described above, an anionic surfactant is preferred, and an ester salt of a fatty acid having 5 or more and 18 or less carbon atoms and isethionic acid is more preferred, from the viewpoint of favorable solubility in water, good lather, and the like. An amino acid-based anionic surfactant such as an N-acylamino acid salt or an N-acyl-N-methylamino acid salt is preferred from the viewpoint of low irritation.

The content of the surfactant in the cleansing composition according to the second embodiment of the present invention is preferably 10% by mass or more, more preferably 15% by mass or more, further more preferably 20% by mass or more, from the viewpoint of improvement in foamability. The content is preferably 50% by mass or less, more preferably 45% by mass or less, further more preferably 40% by mass or less, even more preferably 35% by mass or less, from the viewpoint of improvement in foam break. The content of the surfactant in the cleansing composition is preferably 10% by mass or more and 50% by mass or less, more preferably 15% by mass or more and 45% by mass or less, further more preferably 15% by mass or more and 40% by mass or less, even more preferably 20% by mass or more and 35% by mass or less.

In the cleansing composition according to the second embodiment of the present invention, the mass ratio of the total amount of the carbonate and the organic acid to the surfactant [(carbonate + organic acid)/surfactant] is preferably 0.1 or more, more preferably 1.0 or more, further more preferably 1.5 or more, from the viewpoint of foamability, and preferably 20 or less, more preferably 10 or less, further more preferably 5 or less, from the viewpoint of improvement in washing performance. The mass ratio of the total amount of the carbonate and the organic acid to the surfactant [(carbonate + organic acid)/surfactant] is preferably 0.1 or more and 20 or less, more preferably 1.0 or more and 10 or less, further more preferably 1.5 or more and 5 or less.

In the cleansing composition according to the second embodiment of the present invention, the mass ratio of the organic acid to the carbonate [organic acid/carbonate] is preferably 0.05 or more, more preferably 0.1 or more, further more preferably 0.2 or more, and preferably 10 or less, more preferably 5.0 or less, further more preferably 1.0 or less, from the viewpoint of foamability. The mass ratio of the organic acid to the carbonate [organic acid/carbonate] is preferably 0.05 or more and 10 or less, more preferably 0.1 or more and 5.0 or less, further more preferably 0.2 or more and 1.0 or less.

The total amount of the carbonate and the organic acid in the cleansing composition according to the second embodiment of the present invention is preferably 35% by mass or more, more preferably 40% by mass or more, further more preferably 45% by mass or more, from the viewpoint of foamability, and preferably 70% by mass or less, more preferably 65% by mass or less, further more preferably 60% by mass or less, from the viewpoint of improvement in long-lasting foam. The total amount of the carbonate and the organic acid in the cleansing composition is preferably 35% by mass or more and 70% by mass or less, more preferably 40% by mass or more and 65% by mass or less, further more preferably 45% by mass or more and 60% by mass or less.

The total amount of the carbonate, the organic acid, and the surfactant in the cleansing composition according to the second embodiment of the present invention is preferably 50% by mass or more, more preferably 60% by mass or more, further more preferably 70% by mass or more, from the viewpoint of improvement in washing performance and foamability, and preferably 95% by mass or less, more preferably 90% by mass or less, further more preferably 85% by mass or less, from the viewpoint of improvement in long-lasting foam. The total amount of the carbonate, the organic acid, and the surfactant in the cleansing composition according to the second embodiment of the present invention is preferably 50% by mass or more and 95% by mass or less, more preferably 60% by mass or more and 90% by mass or less, further more preferably 70% by mass or more and 85% by mass or less.

### <Moisture absorbent>

The cleansing composition according to the second embodiment of the present invention preferably further contains a moisture absorbent.

The moisture absorbent used in the cleansing composition according to the second embodiment of the present invention is as described in the first embodiment, and its preferred range is also the same.

The content of the moisture absorbent in the cleansing composition according to the second embodiment of the present invention is preferably 0.01% by mass or more, more preferably 0.1% by mass or more, further more preferably 1% by mass or more, even more preferably 2% by mass or more, even more preferably 4% by mass or more, from the viewpoint of improvement in preservation stability. The content is preferably 10% by mass or less, more preferably 9% by mass or less, further more preferably 8% by mass or less, from the viewpoint of improvement in foamability. The content of the moisture absorbent in the cleansing composition is preferably 0.01% by mass or more and 10% by mass or less, more preferably 0.1% by mass or more and 9% by mass or less, further more preferably 1% by mass or more and 8% by mass or less, even more preferably 2% by mass or more and 8% by mass or less, even more preferably 4% by mass or more and 8% by mass or less.

### <Other components>

The cleansing composition according to the second embodiment of the present invention may contain components which are generally used in cleansing compositions as other components, so long as the object of the present invention is not impaired. Examples of such other components include excipients, binders, natural dyes, moisturizers, anti-inflammatory agents, germicides, antiperspirants, antioxidants, fragrances, and mixtures thereof.

The excipient used in the cleansing composition according to the second embodiment of the present invention is as described in the first embodiment, and its preferred range is also the same.

The content of the excipient in the cleansing composition according to the second embodiment of the present invention is preferably 5% by mass or more, more preferably 10% by mass or more, and preferably 50% by mass or less, more preferably 40% by mass or less, further more preferably 30% by mass or less, from the viewpoint of solubility, granulability, foamability, and preservation stability. The content of the excipient in the cleansing composition is preferably 5% by mass or more and 50% by mass or less, more preferably 10% by mass or more and 40% by mass or less, further more preferably 10% by mass or more and 30% by mass or less.

Examples of the binder used in the cleansing composition according to the second embodiment of the present invention include hydroxyethylcellulose, hydroxymethylcellulose, carboxymethylcellulose, xanthan gum, and carrageenan. One or more of these binders can be used. The content of the binder in the cleansing composition according to the second embodiment of the present invention is preferably 0.1% by mass or more, more preferably 0.3% by mass or more, further more preferably 0.5% by mass or more, and preferably 10% by mass or less, more preferably 5% by mass or less, further more preferably 3% by mass or less, from the viewpoint of instant solubility and productivity. The content of the binder in the cleansing composition is preferably 0.1% by mass or more and 10% by mass or less, more preferably 0.3% by mass or more and 5% by mass or less, further more preferably 0.5% by mass or more and 3% by mass or less.

### <Particle sizes of carbonate particles (a), organic acid particles (b), particles (A), and particles (B)>

The median diameter (D50) for the cleansing composition according to the second embodiment of the present invention is as described in the first embodiment.

The median diameters of the carbonate particles (a), the organic acid particles (b), the particles (A), and the particles (B) in the cleansing composition according to the second embodiment of the present invention are as described in the first embodiment, and their preferred ranges are also the same.

The median diameter of an additional starting material component other than the carbonate and the organic acid in the cleansing composition according to the second embodiment of the present invention is preferably equal to or smaller than the median diameters of the carbonate particles (a) and the organic acid particles (b), for use, and is usually 170 µm or smaller, for example, 5 µm or larger and 150 µm or smaller.

In the cleansingcomposition according to the second embodiment of the present invention, the median diameter of the particles (A) is equal to or larger than the median diameter of the carbonate particles (a), and the median diameter of the particles (B) is equal to or larger than the median diameter of the organic acid particles (b).

When the median diameter of the carbonate particles (a) falls within the range of the median diameter of the particles (A), the carbonate particles (a) can be used as they are as the particles (A). Likewise, when the median diameter of the organic acid particles (b) falls within the range of the median diameter of the particles (B), the organic acid particles (b) can be used as they are as the particles (B).

Alternatively, in the case of setting the median diameter of the particles (A) to be larger than that of the carbonate particles (a), the size can be adjusted by, for example, granulation mentioned later. Likewise, in the case of setting the median diameter of the particles (B) to be larger than that of the organic acid particles (b), the size can be adjusted by, for example, granulation mentioned later.

### <Granulated particles>

In the cleansing composition according to the second embodiment of the present invention, the granulation of the particles (A) containing the carbonate and the particles (B) containing the organic acid is also as described in the first embodiment, and its preferred range is also the same.

For the particles (A) and the particles (B), the particles of at least either the particles (A) or the particles (B) are preferably granulated with one or more members selected from the group consisting of components other than the carbonate particles (a) and the organic acid particles (b), for example, a surfactant, an excipient, a water-soluble polymer (a binder and a feel modifier), and a moisture absorbent, particularly preferably with all the components other than the carbonate particles (a) and the organic acid particles (b), from the viewpoint of reducing the amount of a fine powder. Both the particles (A) and the particles (B) are preferably granulated from the viewpoint of more improving preservation stability and from the viewpoint of improving instant solubility, and either the particles (A) or the particles (B) are preferably granulated from the viewpoint of improving productivity.

The particles (A) or the particles (B) in the cleansing composition according to the second embodiment of the present invention preferably contain the surfactant, or both the particles (A) and the particles (B) preferably contain the surfactant. Specifically, the cleansing composition of the present invention may contain the carbonate particles (a) and particles (B) containing the organic acid and the surfactant, may contain particles (A) containing the carbonate and the surfactant and the organic acid particles (b), or may contain particles (A) containing the carbonate and the surfactant and particles (B) containing the organic acid and the surfactant.

The content of a 10 µm or smaller fine powder in the cleansing composition according to the second embodiment of the present invention is as described in the first embodiment, and its preferred range is also the same.

### <Granulation method>

Any of a fluidized-bed granulation method, a stirring granulation method, a tumbling granulation method, and an extrusion granulation method can be used as a granulation method for the particles (A) or the particles (B) of the cleansing composition according to the second embodiment of the present invention. Among those described above, a fluidized-bed granulation method is preferred from the viewpoint of improving the solubility of the cleansing composition in water.

Specifically, in the case of granulating, for example, the particles (A), granulation can be carried out by charging the starting materials other than the organic acid into a fluidized-bed granulator and adding a binder solution of a binder dissolved in water, ethanol, or a mixed liquid thereof to a uniformly mixed powder and granular material. Likewise, in the case of granulating the particles (B), granulation can be carried out by charging the starting materials other than the carbonate into a fluidized-bed granulator and adding a binder solution of a binder dissolved in water, ethanol, or a mixed liquid thereof to a uniformly mixed powder and granular material.

The content (solid content) of the binder in the binder solution is preferably 0.1% by mass or more, more preferably 0.3% by mass or more, further more preferably 0.5% by mass or more, and preferably 10% by mass or less, more preferably 8% by mass or less, further more preferably 5% by mass or less, from the viewpoint of productivity. The content of the binder in the binder solution is preferably from 0.1 to 10% by mass, more preferably from 0.3 to 8% by mass, further more preferably from 0.5 to 5% by mass.

### <Method for producing cleansing composition>

The method for producing the cleansing composition according to the second embodiment of the present invention contains a step of, by using carbonate particles (a) having a median diameter of 170 µm or smaller and organic acid particles (b) having a median diameter of 170 µm or smaller as starting materials, granulating at least either the carbonate particles (a) or the organic acid particles (b) to prepare granulated particles having a median diameter of 80 µm or larger (hereinafter, also referred to as a "granulated particle preparation step").

Carbonate particles (a) and organic acid particles (b) having a median diameter of 170 µm or smaller are used as starting material particles, and at least either them is granulated so as to be 80 µm or larger, whereby preservation stability can be improved while the cleansing composition thus produced is excellent in solubility, exhibits reduced roughness in texture, and offers favorable feeling in use.

The granulated particle preparation step can be performed by the granulation method mentioned above.

The granulation of at least either the carbonate particles (a) or the organic acid particles (b) includes the case of separately granulating both the carbonate particles (a) and the organic acid particles (b) and the case of granulating only either the carbonate particles (a) or the organic acid particles (b). Specifically, either the carbonate particles (a) or the organic acid particles (b) may be used as starting material particles without being granulated, or the carbonate particles (a) and the organic acid particles (b) may be granulated and used as granulated particles (A) of the carbonate and granulated particles (B) of the organic acid, respectively. Both the carbonate particles (a) and the organic acid particles (b) are preferably separately granulated from the viewpoint of more improving preservation stability and from the viewpoint of improving instant solubility. Only either the carbonate particles (a) or the organic acid particles (b) are preferably granulated from the viewpoint of improving productivity.

After the granulated particle preparation step, the granulated particles (particles (A) and particles (B)) in the case of separately granulating the carbonate particles (a) and the organic acid particles (b), or the granulated particles and ungranulated particles in the case of granulating either the carbonate particles (a) or the organic acid particles (b) can be mixed by a known means to produce a cleansing composition.

### <Method for using cleansing composition>

The cleansing composition according to the second embodiment of the present invention can be suitably used for washing, for example, the face, hair, or the body.

The cleansing composition of the present invention is a foamable cleansing composition which starts to foam spontaneously by the addition of water. Hence, the composition can be conveniently utilized because the composition makes quick lather with the surfactant-containing composition and eliminates the need of a special procedure of making lather, which requires time and labor.

The method for using the cleansing composition according to the second embodiment of the present invention is performed, for example, by putting the cleansing composition on the palm, adding water to the cleansing composition, thoroughly blending the water into the cleansing composition on the palm, allowing the cleansing composition to foam spontaneously, then lightly making lather, and applying the foam to a recipient such as the face, hair, or the whole body for washing.

The amount of the water to be added to the cleansing composition according to the second embodiment of the present invention is preferably 1/30 or more, more preferably 1/25 or more, further more preferably 1/20 or more, and preferably 1/0.5 or less, more preferably 1/1 or less, further more preferably 1/2 or less, in terms of the mass ratio between the cleansing composition and the water to be added (cleansing composition/water) from the viewpoint of improving the solubility of the cleansing composition and also improving foamability.

The temperature of the water to be added to the cleansing composition according to the second embodiment of the present invention is preferably 15°C or higher and 50°C or lower, more preferably 15°C or higher and 45°C or lower, further more preferably 20°C or higher and 45°C or lower, though it is not particularly limited.

The cleansing composition according to the second embodiment of the present invention is applied to the skin within preferably 5 minutes, more preferably 3 minutes, further more preferably 2 minutes, even more preferably 1.5 minutes, after addition of water from the viewpoint of the retention of foam.

The cleansing composition according to the second embodiment of the present invention can be suitably used in a face care product such as a face wash or a body care product such as a hand soap or a body soap.

A foamable cleansing product obtained using the cleansing composition of the present invention can be enclosed in a packaging material and provided. The cleansing composition of the present invention is excellent in preservation stability in the form of a product enclosed in a packaging material because the composition, even when enclosed and preserved in the packaging material, is unlikely to cause the swelling of the packaging material ascribable to the generation of carbonic acid gas.

The shape of the packaging material is not particularly limited so long as it has a structure where the cleansing composition can be enclosed. Examples thereof include bag and bottle shapes. Among them, a bag-shaped packaging material is preferred.

A material constituting the packaging material is not particularly limited so long as the cleansing composition can be enclosed therein. For example, a resin film, or a laminated film of an inorganic thin film of a metal or a metal oxide laminated on a resin film can be used for a bag-shaped packaging material.

Hereinafter, the present invention further discloses the following <1> to <21> in relation to the first embodiment mentioned above.
<1> A carbonic acid foaming composition containing a carbonate and an organic acid, wherein
   the carbonic acid foaming composition contains particles (A) containing the carbonate and particles (B) containing the organic acid,
   the particles (A) are particles containing no organic acid, and the particles (B) are particles containing no carbonate,
   a median diameter of carbonate particles (a) as a starting material of the particles (A) is 170 µm or smaller, and a median diameter of organic acid particles (b) as a starting material of the particles (B) is 500 µm or smaller,
   a median diameter of particles of at least either the particles (A) or the particles (B) is 80 µm or larger,
   the median diameter of the particles (A) is equal to or larger than the median diameter of the carbonate particles (a), and the median diameter of the particles (B) is equal to or larger than the median diameter of the organic acid particles (b).
<2> The carbonic acid foaming composition according to <1>, wherein the particles of at least either the particles (A) or the particles (B) contain granulated particles obtained by granulating starting material particles.
<3> The carbonic acid foaming composition according to <1> or <2>, further containing a moisture absorbent.
<4> The carbonic acid foaming composition according to any one of <1> to <3>, wherein the organic acid contains one or more members selected from the group consisting of citric acid, succinic acid, tartaric acid, and ascorbic acid.
<5> The carbonic acid foaming composition according to any one of <1> to <4>, wherein the carbonate contains one or more members selected from the group consisting of sodium carbonate and sodium bicarbonate.
<6> The carbonic acid foaming composition according to any one of <3> to <5>, wherein the moisture absorbent is magnesium oxide.
<7> The carbonic acid foaming composition according to any one of <3> to <6>, wherein the particles (A) or the particles (B) are granulated particles further containing the moisture absorbent.
<8> The carbonic acid foaming composition according to any one of <1> to <7>, wherein the particles (A) are granulated particles further containing an excipient.
<9> The carbonic acid foaming composition according to any one of <1> to <7>, wherein the particles (B) are granulated particles further containing an excipient.
<10> The carbonic acid foaming composition according to any one of <1> to <7>, wherein both the particles (A) and the particles (B) are granulated particles further containing an excipient.
<11> A method for producing the carbonic acid foaming composition according to any one of <1> to <10>, containing a step of,
   by using carbonate particles (a) having a median diameter of 170 µm or smaller and organic acid particles (b) having a median diameter of 500 µm or smaller as starting materials, granulating at least either the carbonate particles (a) or the organic acid particles (b) to prepare granulated particles having a median diameter of 80 µm or larger.
<12> The carbonic acid foaming composition according to any one of <1> to <10> or the method for producing a carbonic acid foaming composition according to <11>, wherein the median diameter of the particles (a) is 10 µm or larger and 150 µm or smaller, and the median diameter of the particles (b) is 10 µm or larger and 350 µm or smaller.
<13> The carbonic acid foaming composition according to any one of <1> to <10> or the method for producing a carbonic acid foaming composition according to <11> or <12>, wherein the median diameter of the particles of at least either the particles (A) or the particles (B) is 90 µm or larger.
<14> The carbonic acid foaming composition according to any one of <2> to <10> or the method for producing a carbonic acid foaming composition according to any one of <11> to <13>, wherein a median diameter of the granulated particles is 110 µm or larger and 350 µm or smaller.
<15> The carbonic acid foaming composition according to any one of <1> to <10> or the method for producing a carbonic acid foaming composition according to any one of <11> to <14>, wherein a content of the carbonate in the carbonic acid foaming composition is 15% by mass or more and 65% by mass or less.
<16> The carbonic acid foaming composition according to any one of <1> to <10> or the method for producing a carbonic acid foaming composition according to any one of <11> to <15>, wherein a content of the organic acid in the carbonic acid foaming composition is 5% by mass or more and 60% by mass or less.
<17> The carbonic acid foaming composition according to any one of <1> to <10> or the method for producing a carbonic acid foaming composition according to any one of <11> to <16>, wherein a mass ratio of the organic acid to the carbonate [organic acid/carbonate] is 0.05 or more and 10 or less.
<18> The carbonic acid foaming composition according to any one of <1> to <10> or the method for producing a carbonic acid foaming composition according to any one of <11> to <17>, wherein a total amount of the carbonate and the organic acid in the carbonic acid foaming composition is from 50 to 90% by mass.
<19> The carbonic acid foaming composition according to any one of <3> to <10> or the method for producing a carbonic acid foaming composition according to any one of <11> to <18>, wherein a content of the moisture absorbent in the carbonic acid foaming composition is from 0.01 to 15% by mass.
<20> The carbonic acid foaming composition according to any one of <1> to <10> or the method for producing a carbonic acid foaming composition according to any one of <11> to <19>, wherein a value of a coefficient of variation, CV, represented by the following expression (1) in the particles (a) and the particles (b) is 95% or less: 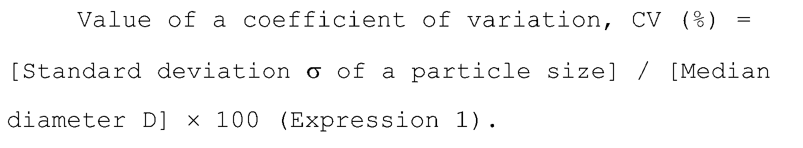 Value of a coefficient of variation, CV (%) = [Standard deviation σ of a particle size] / [Median diameter D] × 100 (Expression 1)
<21> The carbonic acid foaming composition according to any one of <1> to <10> or the method for producing a carbonic acid foaming composition according to any one of <11> to <20>, wherein a content of 10 µm or smaller particles is 0.001% by mass or more and 3.5% by mass or less in all particles constituting the carbonic acid foaming composition.

The present invention further discloses the following <31> to <51> in relation to the second embodiment mentioned above.
<31> A cleansing composition containing a carbonate, an organic acid, and a surfactant, wherein
   the cleansing composition contains particles (A) containing the carbonate and particles (B) containing the organic acid,
   the particles (A) are particles containing no organic acid, and the particles (B) are particles containing no carbonate,
   a median diameter of carbonate particles (a) as a starting material of the particles (A), and a median diameter of organic acid particles (b) as a starting material of the particles (B) are 170 µm or smaller,
   a median diameter of particles of at least either the particles (A) or the particles (B) is 80 µm or larger,
   the median diameter of the particles (A) is equal to or larger than the median diameter of the carbonate particles (a), and the median diameter of the particles (B) is equal to or larger than the median diameter of the organic acid particles (b).
<32> The cleansing composition according to <31>, wherein the particles of at least either the particles (A) or the particles (B) contain granulated particles obtained by granulating starting material particles.
<33> The cleansing composition according to <31> or <32>, further containing a moisture absorbent.
<34> The cleansing composition according to any one of <31> to <33>, wherein the organic acid contains one or more members selected from the group consisting of citric acid, succinic acid, tartaric acid, and ascorbic acid.
<35> The cleansing composition according to any one of <31> to <34>, wherein the carbonate contains one or more members selected from the group consisting of sodium carbonate and sodium bicarbonate.
<36> The cleansing composition according to any one of <33> to <35>, wherein the moisture absorbent is magnesium oxide.
<37> The cleansing composition according to any one of <33> to <36>, wherein the particles (A) or the particles (B) are granulated particles further containing the surfactant and the moisture absorbent.
<38> The cleansing composition according to any one of <31> to <37>, wherein a content of the carbonate is 10% by mass or more and 60% by mass or less.
<39> The cleansing composition according to any one of <31> to <38>, wherein a content of the organic acid is 5% by mass or more and 60% by mass or less.
<40> The cleansing composition according to any one of <31> to <39>, wherein a content of the surfactant is 10% by mass or more and 50% by mass or less.
<41> The cleansing composition according to any one of <31> to <40>, wherein a mass ratio of a total amount of the carbonate and the organic acid to the surfactant [(carbonate + organic acid)/surfactant] is 0.1 or more and 20 or less.
<42> The cleansing composition according to any one of <31> to <41>, wherein a mass ratio of the organic acid to the carbonate [organic acid/carbonate] is 0.05 or more and 10 or less.
<43> The cleansing composition according to any one of <31> to <42>, wherein a total amount of the carbonate and the organic acid is 35% by mass or more and 70% by mass or less.
<44> The cleansing composition according to any one of <31> to <43>, wherein the median diameter of the carbonate particles (a) as a starting material of the particles (A), and the median diameter of the organic acid particles (b) as a starting material of the particles (B) are 20 µm or larger and 120 µm or smaller, and the median diameter of the particles of at least either the particles (A) or the particles (B) is 90 µm or larger and 400 µm or smaller.
<45> The cleansing composition according to any one of <31> to <44>, wherein the content of the carbonate is 20% by mass or more and 50% by mass or less, and the content of the organic acid is 7% by mass or more and 30% by mass or less.
<46> The cleansing composition according to any one of <31> to <45>, wherein the content of the surfactant is 15% by mass or more and 45% by mass or less, and the mass ratio of the total amount of the carbonate and the organic acid to the surfactant [(carbonate + organic acid)/surfactant] is 1.0 or more and 10 or less.
<47> The cleansing composition according to any one of <31> to <46>, wherein the particles (A) contain the surfactant.
<48> The cleansing composition according to any one of <31> to <46>, wherein the particles (B) contain the surfactant.
<49> The cleansing composition according to any one of <31> to <46>, wherein both the particles (A) and the particles (B) contain the surfactant.
<50> A method for producing the cleansing composition according to any one of <31> to <49>, containing a step of,
   by using carbonate particles (a) having a median diameter of 170 µm or smaller and organic acid particles (b) having a median diameter of 170 µm or smaller as starting materials, granulating at least either the carbonate particles (a) or the organic acid particles (b) to prepare granulated particles having a median diameter of 80 µm or larger.
<51> The method for producing a cleansing composition according to <50>, wherein the median diameters of the carbonate particles (a), and the organic acid particles (b) are 20 µm or larger and 120 µm or smaller, and the median diameter of the granulated particles is 90 µm or larger and 400 µm or smaller.

### Examples

Hereinafter, the present invention will be described with reference to Examples. However, the present invention is not limited by the scope of Examples. Measurement in the present Examples was performed by the following methods.

### [Method for measuring median diameter and CV value]

For the particle sizes of carbonate particles (a), organic acid particles (b), particles (A), and particles (B), a median diameter and a CV value were measured with CAMSIZER XT (particle size measurement apparatus, manufactured by Retsch GmbH) using 3 g each of the particles. In the case of granulation products of the particles (A) and the particles (B), each measurement was performed after granulation.

### [Method for measuring content (proportion by number) of granulated particles in particles]

A powder or granular cleansing composition was sieved through a sieve with an opening of 100 µm, and particles on the sieve were collected with a very small spatula. The collected particles were spread over a 5 mm × 5 mm carbon tape and observed under Hitachi Tabletop Microscope Miniscope TM3030 (manufactured by Hitachi High-Tech Corp.). Arbitrary 20 grains were observed, and the content (proportion by number) of aggregates as the granulated particles was calculated.

### [Preparation of mixed surfactant]

300 g of sodium N-myristoyl-L-glutamate (Amisoft MS manufactured by Ajinomoto Co., Inc.) and 291.5 g of sodium N-lauroyl-L-glutamate (Amisoft LS manufactured by Ajinomoto Co., Inc.) were mixed at room temperature to give a mixed surfactant.

### <First embodiment>

### [Example 1-1]

A carbonic acid foaming composition was obtained in accordance with the formulation shown in Table 1.

A fluidized-bed granulator (FD-MP-01E manufactured by Powrex Corp.) was charged with 158.6 g of citric acid (Citric Anhydride 60 manufactured by Iwata Chemical Co., Ltd.), 39.5 g of magnesium oxide (manufactured by Kyowa Chemical Industry Co., Ltd.), 84.7 g of talc (SW-K4 manufactured by Asada Milling Co., Ltd.), and 16.3 g of cationized hydroxyethylcellulose (SOFTCAT Polymer SL-30 manufactured by The Dow Chemical Company), which were then granulated under conditions involving an air flow of 0.2 m³/min and a suction temperature of 80°C while 90 g (solid content: 0.9 g) of an aqueous solution of 1.0% carboxymethylcellulose (manufactured by Nippon Paper Industries Co., Ltd., SUNROSE; hereinafter, also referred to as CMC) was added at a rate of 4 g/min, to give a granulation product having a median diameter of 175 µm. The obtained granulation product and 264.3 g of sodium bicarbonate (hereinafter, also referred to as baking soda; manufactured by AGC Inc.) were added to a bag and manually mixed until uniform in a clear vinyl bag to prepare a carbonic acid foaming composition.

The obtained carbonic acid foaming composition was evaluated for each item in accordance with a method given below. The results are shown in Table 1.

### [Examples 1-2 and 1-3]

Each carbonic acid foaming composition was obtained by the same method as in Example 1-1 except that the formulation was as shown in Table 1.

The obtained carbonic acid foaming composition was evaluated for each item in accordance with a method given below. The results are shown in Table 1.

### [Example 1-4]

Sodium bicarbonate was granulated in accordance with the formulation shown in Table 1. A fluidized-bed granulator (FD-MP-01E manufactured by Powrex Corp.) was charged with 195.3 g of sodium bicarbonate (manufactured by AGC Inc.), 29.2 g of magnesium oxide (manufactured by Kyowa Chemical Industry Co., Ltd.), 62.6 g of talc (SW-K4 manufactured by Asada Milling Co., Ltd.), and 12.0 g of cationized hydroxyethylcellulose (SOFTCAT Polymer SL-30 manufactured by The Dow Chemical Company), which were then granulated under the same conditions as in Example 1-1 while 66 g (solid content: 0.7 g) of an aqueous solution of 1.0% CMC was added at a rate of 4 g/min, to give a granulation product having a median diameter of 145 µm. The obtained granulation product and 117.2 g of citric acid (Citric Anhydride 60 manufactured by Iwata Chemical Co., Ltd.) were manually mixed until uniform in a clear vinyl bag to prepare a carbonic acid foaming composition.

The obtained carbonic acid foaming composition was evaluated for each item in accordance with a method given below. The results are shown in Table 1.

### [Example 1-5]

A carbonic acid foaming composition was obtained by the same method as in Example 1-1 except that citric acid (Fine Citric Acid Powder manufactured by Iwata Chemical Co., Ltd.) was used.

The obtained carbonic acid foaming composition was evaluated for each item in accordance with a method given below. The results are shown in Table 1.

### [Example 1-6]

A carbonic acid foaming composition was obtained by the same method as in Example 1-1 except that 142.9 g of citric acid (Citric Anhydride 60 manufactured by Iwata Chemical Co., Ltd.), 37.6 g of magnesium oxide (manufactured by Kyowa Chemical Industry Co., Ltd.), 75.8 g of talc (SW-K4 manufactured by Asada Milling Co., Ltd.), 15.6 g of cationized hydroxyethylcellulose (SOFTCAT Polymer SL-30 manufactured by The Dow Chemical Company), and 26.9 g of sodium cocoyl isethionate (JODAPONLACI manufactured by BASF ED) were used.

The obtained carbonic acid foaming composition was evaluated for each item in accordance with a method given below. The results are shown in Table 1.

### [Comparative Examples 1-1 to 1-3]

Each carbonic acid foaming composition was obtained in accordance with the formulation shown in Table 2.

In Comparative Examples 1-1 to 1-3, all components were added to a bag without being granulated, and manually mixed until uniform in a clear vinyl bag to give a carbonic acid foaming composition.

The obtained carbonic acid foaming composition was evaluated for each item in accordance with a method given below. The results are shown in Table 2.

### [Foamability (without addition of water)]

1 g of each carbonic acid foaming composition was put on the palm, and 6 g of a commercially available shampoo liquid (Kao Corp., Essential The Beauty) was added to the carbonic acid foaming composition. The shampoo liquid was thoroughly blended into the carbonic acid foaming composition on the palm and allowed to foam spontaneously. Then (after approximately 10 seconds from the addition of the shampoo liquid), these were mixed by fingers of the other hand to make lather. Then, foamability in making lather within 3 minutes from the addition of the shampoo liquid in a state in which the operation of making lather was continued was evaluated by three specialist panels in accordance with the following criteria and determined by the discussion among the specialist panels.
1: A palmful of foam was made in shorter than 30 seconds
2: A palmful of foam was made in 30 seconds or longer and shorter than 1 minute
3: A palmful of foam was made in 1 minute or longer and shorter than 2 minutes
4: A palmful of foam was made in 2 minutes or longer and shorter than 3 minutes
5: Making a palmful of foam took 3 minutes or longer [Foamability (with addition of water)]

1 g of each carbonic acid foaming composition was put on the palm, and 5 g of water of 42°C was added to the carbonic acid foaming composition. The water was thoroughly blended into the carbonic acid foaming composition on the palm and allowed to foam spontaneously. Then (after a lapse of approximately 10 seconds from the addition of the water), 6 g of a commercially available shampoo liquid (Kao Corp., Essential The Beauty) was added thereto, and the shampoo liquid was thoroughly blended into the carbonic acid foaming composition on the palm and allowed to foam spontaneously. Then, these were mixed by fingers of the other hand to make lather. Then, foamability in making lather within 3 minutes from the addition of the water in a state in which the operation of making lather was continued was evaluated by three specialist panels in accordance with the following criteria and determined by the discussion among the specialist panels.
1: A palmful of foam was made in shorter than 30 seconds
2: A palmful of foam was made in 30 seconds or longer and shorter than 1 minute
3: A palmful of foam was made in 1 minute or longer and shorter than 2 minutes
4: A palmful of foam was made in 2 minutes or longer and shorter than 3 minutes
5: Making a palmful of foam took 3 minutes or longer

### [Preservation stability]

3.9 g of each carbonic acid foaming composition was enclosed in an 80 mm × 50 mm × 18 mm aluminum packaging material at 50°C under 50% RH and stored at 50°C for 4 weeks. The amount of the aluminum packaging material swelling was measured before and after the storage. Specifically, a water bath filled with water of 25°C was placed on a scale, and its weight was measured. Next, the aluminum packaging material in which the carbonic acid foaming composition was enclosed was completely submerged in the water bath, and its mass was measured. The difference in mass between before and after the aluminum packaging material was submerged in water was converted to a volume with the density of water defined as 1.0 g/ml in accordance with the principle of Archimedes, and this volume was regarded as the volume of the aluminum packaging material before storage. Then, the same measurement as above was performed after storage for 4 weeks to determine the volume of the aluminum packaging material after the storage. The amount of the aluminum packaging material swelling after the storage was determined from the difference in volume of the aluminum packaging material between before and after the storage. A smaller amount of the packaging material swelling indicates better preservation stability.

### [Feeling in use (texture/without addition of water)]

1 g of each carbonic acid foaming composition was put on the palm, and 6 g of a commercially available shampoo liquid (Kao Corp., Essential The Beauty) was added to the carbonic acid foaming composition. The shampoo liquid was thoroughly blended into the carbonic acid foaming composition on the palm and allowed to foam spontaneously. Then, these were mixed by fingers of the other hand to make lather.

Then, texture in making lather within 3 minutes from the addition of the shampoo liquid to the carbonic acid foaming composition in a state in which the operation of making lather was continued was evaluated by three specialist panels in accordance with the following criteria and determined by the discussion among the specialist panels.
1: No roughness was felt from the beginning
2: Roughness disappeared at the initial stage of making lather, though felt first
3: Roughness disappeared during making lather, though felt first
4: Roughness disappeared after making lather, though felt
5: Roughness was felt and did not disappear even after making lather
(The phrase "initial stage of making lather" refers to within 1 minute from the addition of the shampoo liquid to the carbonic acid foaming composition; the phrase "during making lather" refers to longer than 1 minute and within 2 minutes from the addition; and the phrase "after making lather" refers to longer than 2 minutes and within 3 minutes from the addition.)

### [Feeling in use (texture/with addition of water)]

1 g of each carbonic acid foaming composition was put on the palm, and 5 g of water of 42°C was added to the carbonic acid foaming composition. The water was thoroughly blended into the carbonic acid foaming composition on the palm and allowed to foam spontaneously. Then (after a lapse of approximately 10 seconds from the addition of the water), 6 g of a commercially available shampoo liquid (Kao Corp., Essential The Beauty) was added thereto, and the shampoo liquid was thoroughly blended into the carbonic acid foaming composition on the palm and allowed to foam spontaneously. Then, these were mixed by fingers of the other hand to make lather. Then, texture in making lather within 3 minutes from the addition of the water in a state in which the operation of making lather was continued was evaluated by three specialist panels in accordance with the following criteria and determined by the discussion among the specialist panels.
1: No roughness was felt from the beginning
2: Roughness disappeared at the initial stage of making lather, though felt first
3: Roughness disappeared during making lather, though felt first
4: Roughness disappeared after making lather, though felt
5: Roughness was felt and did not disappear even after making lather
(The phrase "initial stage of making lather" refers to within 1 minute from the addition of the water to the carbonic acid foaming composition; the phrase "during making lather" refers to longer than 1 minute and within 2 minutes from the addition; and the phrase "after making lather" refers to longer than 2 minutes and within 3 minutes from the addition.)

### [Table 1]

**Table 1**

| | | | | | Example | | | | | |
|---|---|---|---|---|---|---|---|---|---|---|
| | | | | | 1-1 | 1-2 | 1-3 | 1-4 | 1-5 | 6 |
| Baking soda particle size (median diameter) | | | Carbonate particle (a) | [µm] | 100 | 57 | 160 | 57 | 100 | 100 |
| | | | CV value of (a) | [%] | 71.7 | 60.0 | 65.0 | 60.0 | 71.7 | 71.7 |
| | | | Particles (A) containing carbonate | [µm] | 100 | 57 | 160 | 145 | 100 | 100 |
| Citric acid particle size (median diameter) | | | Organic acid particle (b) | [µm] | 38 | 38 | 38 | 38 | 326 | 38 |
| | | | CV value of (b) | [%] | 63.6 | 63.6 | 63.6 | 63.6 | 23.3 | 63.6 |
| | | | Particles (B) containing organic acid | [µm] | 175 | 175 | 175 | 38 | 384 | 160 |
| Granulated state*1 | | | | | Organic acid granulated | Organic acid granulated | Organic acid granulated | Carbonate granulated | Organic acid granulated | Organic acid granulated |
| Starting material | Carbonate | | Baking soda | [% by mass] | 46.8 | 46.8 | 46.8 | 46.8 | 46.8 | 44.3 |
| | Organic acid | | Citric acid | [% by mass] | 28.1 | 28.1 | 28.1 | 28.1 | 28.1 | 26.6 |
| | Excipient | | Talc | [% by mass] | 15.0 | 15.0 | 15.0 | 15.0 | 15.0 | 14.1 |
| | Water-soluble polymer | Feel modifier | Cationized hydroxycellulose*2 | [% by mass] | 2.9 | 2.9 | 2.9 | 2.9 | 2.9 | 2.9 |
| | | Binder | CMC (solid content)*3 | [% by mass] | 0.2 | 0.2 | 0.2 | 0.2 | 0.2 | 0.2 |
| | Moisture absorbent | | Magnesium oxide | [% by mass] | 7.0 | 7.0 | 7.0 | 7.0 | 7.0 | 7.0 |
| | Surfactant | | Na cocoyl isethionate | [% by mass] | 0.0 | 0.0 | 0.0 | 0.0 | 0.0 | 5.0 |
| | Total | | | [% by mass] | 100.0 | 100.0 | 100.0 | 100.0 | 100.0 | 100.0 |
| Carbonate + organic acid | | | | [% by mass] | 74.9 | 74.9 | 74.9 | 74.9 | 74.9 | 70.8 |
| [Organic acid/carbonate] | | | | [mass ratio] | 0.60 | 0.60 | 0.60 | 0.60 | 0.60 | 0.60 |
| Evaluation | Preservation stability | | | [ml] | 0.2 | 0.4 | 0.2 | 2.1 | 0.1 | 0.1 |
| | Feeling in use(texture/without addition of water) | | | [-] | 1 | 1 | 2 | 1 | 2 | 1 |
| | Feeling in use(texture/with addition of water) | | | [-] | 1 | 1 | 3 | 1 | 2 | 1 |
| | Foamability (without addition of water) | | | [-] | 1 | 1 | 1 | 1 | 2 | 1 |
| | Foamability (with addition of water) | | | [-] | 1 | 1 | 1 | 1 | 1 | 1 |

### [Table 2]

**Table 2**

| | | | | | Comparative Example | | |
|---|---|---|---|---|---|---|---|
| | | | | | 1-1 | 1-2 | 1-3 |
| Baking soda particle size (median diameter) | | | Carbonate particle (a) | [µm] | 57 | 100 | 176 |
| | | | CV value of (a) | [%] | 60.0 | 71.7 | 65.0 |
| | | | Particles (A) containing carbonate | [%ₗm] | 57 | 100 | 176 |
| Citric acid particle size (median diameter) | | | Organic acid particle (b) | [µm] | 38 | 720 | 38 |
| | | | CV value of (b) | [%] | 63.6 | 36.3 | 63.6 |
| | | | Particles (B) containing organic acid | [µm] | 38 | 720 | 38 |
| Granulated state*1 | | | | | Ungranulated | Ungranulated | Ungranulated |
| Starting material | Carbonate | | Baking soda | [% by mass] | 46.8 | 46.8 | 46.8 |
| | Organic acid | | Citric acid | [% by mass] | 28.1 | 28.1 | 28.1 |
| | Excipient | | Talc | [% by mass] | 15.0 | 15.0 | 15.0 |
| | Water-soluble polymer | Feel modifier | Cationized hydroxycellulose*2 | [% by mass] | 2.9 | 2.9 | 2.9 |
| | | Binder | CMC (solid content)*3 | [% by mass] | 0.2 | 0.2 | 0.2 |
| | Moisture absorbent | | Magnesium oxide | [% by mass] | 7.0 | 7.0 | 7.0 |
| | Surfactant | | Na cocoyl isethionate | [% by mass] | 0.0 | 0.0 | 0.0 |
| | Total | | | [% by mass] | 100.0 | 100.0 | 100.0 |
| Carbonate + organic acid | | | | [% by mass] | 74.9 | 74.9 | 74.9 |
| [Organic acid/carbonate] | | | | [mass ratio] | 0.60 | 0.60 | 0.60 |
| Evaluation | Preservation stability | | | [ml] | 50 | 0.1 | 1.8 |
| | Feeling in use(texture/without addition of water) | | | [-] | 1 | 4 | 4 |
| | Feeling in use(texture/with addition of water) | | | [-] | 1 | 5 | 5 |
| | Foamability (without addition of water) | | | [-] | 1 | 3 | 1 |
| | Foamability (with addition of water) | | | [-] | 1 | 2 | 1 |

*1 "Organic acid granulated" described in the section "Granulated state" in Tables 1 and 2, means that the organic acid and all the components (excipient, water-soluble polymer, and moisture absorbent) other than the carbonate were granulated. The resulting granulated particles correspond to the particles (B) containing the organic acid of the present invention. In the case of "Organic acid granulated", the carbonate (baking soda) was used as ungranulated starting material particles (which correspond to the particles (A) containing the carbonate).
   "Carbonate granulated" means that the carbonate and all the components (excipient, water-soluble polymer, and moisture absorbent) other than the organic acid were granulated. The resulting granulated particles correspond to the particles (A) containing the carbonate of the present invention. In the case of "Carbonate granulated", the organic acid (citric acid) was used as ungranulated starting material particles (which correspond to the particles (B) containing the organic acid).
   "Ungranulated" refers to a state in which all the powder components were used without being granulated.
*2 SOFTCAT Polymer SL-30 manufactured by The Dow Chemical Company
*3 SUNROSE manufactured by Nippon Paper Industries Co., Ltd.

It is evident from Tables 1 and 2 that the carbonic acid foaming compositions of the present Examples are excellent in preservation stability and excellent in texture.

All the shampoos to which the carbonic acid foaming compositions of the present Examples were applied had favorable foamability, foam break, and long-lasting foam.

### <Second embodiment>

### [Example 2-1]

A cleansing composition was obtained in accordance with the formulation shown in Table 3.

A fluidized-bed granulator (FD-MP-01E manufactured by Powrex Corp.) was charged with 67.4 g of citric acid (Citric Anhydride 60 manufactured by Iwata Chemical Co., Ltd.), 118.3 g of a mixed surfactant, 25.2 g of magnesium oxide (manufactured by Kyowa Chemical Industry Co., Ltd.), and 84.0 g of talc (SW-K4 manufactured by Asada Milling Co., Ltd.), which were then granulated under conditions involving an air flow of 0.3 m³/min and a suction temperature of 80°C while 314 g (solid content: 5.0 g) of an aqueous solution of 1.6% carrageenan was added at a rate of 5 g/min, to give a granulation product having a median diameter of 172 µm. The obtained granulation product and 203 g of sodium bicarbonate (hereinafter, also referred to as baking soda; manufactured by AGC Inc.) were added to a clear vinyl bag and manually mixed until uniform in the bag to prepare a cleansing composition.

The obtained cleansing composition was evaluated for each item in accordance with a method given below. The results are shown in Table 3.

### [Examples 2-2, 2-3 and 2-5 to 2-8]

Each cleansing composition was obtained by the same method as in Example 2-1 except that the formulation was as shown in Table 3.

The obtained cleansing composition was evaluated for each item in accordance with a method given below. The results are shown in Table 3.

### [Example 2-4]

Sodium bicarbonate was granulated in accordance with the formulation shown in Table 3. A fluidized-bed granulator (FD-MP-01E manufactured by Powrex Corp.) was charged with 139.9 g of sodium bicarbonate (manufactured by AGC Inc.), 81.5 g of a mixed surfactant, 17.3 g of magnesium oxide (manufactured by Kyowa Chemical Industry Co., Ltd.), and 57.8 g of talc (SW-K4 manufactured by Asada Milling Co., Ltd.), which were then granulated under the same conditions as in Example 2-1 while 216 g (solid content: 3.5 g) of an aqueous solution of 1.6% carrageenan was added at a rate of 5 g/min, to give a granulation product having a median diameter of 127 µm. The obtained granulation product and 46.6 g of citric acid were manually mixed until uniform in a clear vinyl bag to prepare a cleansing composition.

The obtained cleansing composition was evaluated for each item in accordance with a method given below. The results are shown in Table 3.

### [Comparative Examples 2-1 to 2-3]

Each cleansing composition was obtained in accordance with the formulation shown in Table 4.

In Comparative Examples 2-1 to 2-3, all components were added to a bag without being granulated, and manually mixed in the bag to give a cleansing composition.

The obtained cleansing composition was evaluated for each item in accordance with a method given below. The results are shown in Table 4.

### [Preservation stability]

3.9 g of each cleansing composition was enclosed in an 80 mm × 50 mm × 18 mm aluminum packaging material at 50°C under 50% RH and stored at 50°C for 4 weeks. The amount of the aluminum packaging material swelling was measured before and after the storage. Specifically, a water bath filled with water of 25°C was placed on a scale, and its weight was measured. Next, the aluminum packaging material in which the cleansing composition was enclosed was completely submerged in the water bath, and its mass was measured. The difference in mass between before and after the aluminum packaging material was submerged in water was converted to a volume with the density of water defined as 1.0 g/ml in accordance with the principle of Archimedes, and this volume was regarded as the volume of the aluminum packaging material before storage. Then, the same measurement as above was performed after storage for 4 weeks to determine the volume of the aluminum packaging material after the storage. The amount of the aluminum packaging material swelling after the storage was determined from the difference in volume of the aluminum packaging material between before and after the storage. A smaller amount of the packaging material swelling indicates better preservation stability.

### [texture]

1 g of each cleansing composition was put on the palm, and 10 g of water of 42°C was added dropwise to the cleansing composition. The water was thoroughly blended into the cleansing composition on the palm and allowed to foam spontaneously. Then (after a lapse of approximately 10 seconds from the dropwise addition of the water), both the palms were brought together to make lather. Then, texture in making lather within 3 minutes from the dropwise addition of the water in a state in which the operation of making lather was continued was evaluated by three specialist panels in accordance with the following criteria and determined by the discussion among the specialist panels.
1: No roughness was felt from the beginning
2: Roughness disappeared at the initial stage of making lather, though felt first
3: Roughness disappeared during making lather, though felt first
4: Roughness disappeared after making lather, though felt
5: Roughness was felt and did not disappear even after making lather
(The phrase "initial stage of making lather" refers to within 1 minute from the dropwise addition of the water to the cleansing composition; the phrase "during making lather" refers to longer than 1 minute and within 2 minutes from the dropwise addition; and the phrase "after making lather" refers to longer than 2 minutes and within 3 minutes from the dropwise addition.)

### [Table 3]

**Table 3**

| | | | | Example | | | | | | | |
|---|---|---|---|---|---|---|---|---|---|---|---|
| | | | | 2-1 | 2-2 | 2-3 | 2-4 | 2-5 | 2-6 | 2-7 | 2-8 |
| Baking soda particle size*3 | | Carbonate particle (a) | [µm] | 100 | 100 | 100 | 41 | 41 | 147 | 162 | 41 |
| | | CV value of (a) | [%] | 71.7 | 71.7 | 71.7 | 60.0 | 60.0 | 65.0 | 65.0 | 60.0 |
| | | Particles (A) containing carbonate | [µm] | 100 | 100 | 100 | 127 | 41 | 147 | 162 | 41 |
| Citric acid particle size*3 | | Organic acid particle (b) | [µm] | 38 | 65 | 65 | 38 | 38 | 38 | 38 | 38 |
| | | CV value of (b) | [%] | 63.6 | 91.1 | 91.1 | 63.6 | 63.6 | 63.6 | 63.6 | 63.6 |
| | | Particles (B) containing organic acid | [µm] | 172 | 97 | 81 | 38 | 172 | 172 | 172 | 85 |
| Granulated state*1 | | | | Organic acid granulated | Organic acid granulated | Organic acid granulated | Carbonate granulated | Organic acid granulated | Organic acid granulated | Organic acid granulated | Organic acid granulated |
| Content of granulated particles in particles (proportion by number) | | | [% by number] | 60% | 55% | 50% | 40% | 60% | 60% | 60% | 60% |
| Cleansing composition | Carbonate | Baking soda | [% by mass] | 40.4 | 40.4 | 40.4 | 40.4 | 40.4 | 40.4 | 40.4 | 40.4 |
| | Organic acid | Citric acid | [% by mass] | 13.4 | 13.4 | 13.4 | 13.4 | 13.4 | 13.4 | 13.4 | 13.4 |
| | Surfactant | Mixed surfactant | [% by mass] | 23.5 | 23.5 | 23.5 | 23.5 | 23.5 | 23.5 | 23.5 | 23.5 |
| | Excipient | Talc | [% by mass] | 16.7 | 18.7 | 20.7 | 16.7 | 16.7 | 16.7 | 16.7 | 16.7 |
| | Binder | Carrageenan*2 | [% by mass] | 1.0 | 1.0 | 1.0 | 1.0 | 1.0 | 1.0 | 1.0 | 1.0 |
| | Moisture absorbent | Magnesium oxide | [% by mass] | 5.0 | 3.0 | 1.0 | 5.0 | 5.0 | 5.0 | 5.0 | 5.0 |
| | Total | | [% by mass] | 100.0 | 100.0 | 100.0 | 100.0 | 100.0 | 100.0 | 100.0 | 100.0 |
| | Carbonate + organic acid | | [% by mass] | 53.8 | 53.8 | 53.8 | 53.8 | 53.8 | 53.8 | 53.8 | 53.8 |
| | Carbonate + organic acid + surfactant | | [% by mass] | 77.3 | 77.3 | 77.3 | 77.3 | 77.3 | 77.3 | 77.3 | 77.3 |
| | [Organic acid/carbonate] | | mass ratio | 0.33 | 0.33 | 0.33 | 0.33 | 0.33 | 0.33 | 0.33 | 0.33 |
| | [(Carbonate + organic acid)/Surfactant] | | mass ratio | 2.3 | 2.3 | 2.3 | 2.3 | 2.3 | 2.3 | 2.3 | 2.3 |
| Evaluation | Preservation stability | | [ml] | 0.2 | 2.4 | 4.7 | 4.1 | 4.9 | 0.2 | 0.2 | 7.4 |
| | Texture | | [-] | 1 | 1 | 1 | 1 | 1 | 2 | 3 | 1 |

### [Table 4]

**Table 4**

| | | | | Comparative Example | | |
|---|---|---|---|---|---|---|
| | | | | 2-1 | 2-2 | 2-3 |
| Baking soda particle size*3 | | Carbonate particle (a) | [µm] | 41 | 100 | 180 |
| | | CV value of (a) | [%] | 60.0 | 71.7 | 65.0 |
| | | Particles (A) containing carbonate | [µm] | 41 | 100 | 180 |
| Citric acid particle size*3 | | Organic acid particle (b) | [µm] | 38 | 720 | 38 |
| | | CV value of (b) | [%] | 63.6 | 36.3 | 63.6 |
| | | Particles (B) containing organic acid | [µm] | 38 | 720 | 38 |
| Granulated state*1 | | | | Ungranulated | Ungranulated | Ungranulated |
| Content of granulated particles in particles (proportion by number) | | | [% by number] | 0% | 0% | 0% |
| Cleansing composition | Carbonate | Baking soda | [% by mass] | 40.4 | 40.4 | 40.4 |
| | Organic acid | Citric acid | [% by mass | 13.4 | 13.4 | 13.4 |
| | Surfactant | Mixed surfactant | [% by mass | 23.5 | 23.5 | 23.5 |
| | Excipient | Talc | [% by mass | 16.7 | 16.7 | 16.7 |
| | Binder | Carrageenan*2 | [% by mass | 1.0 | 1.0 | 1.0 |
| | Moisture absorbent | Magnesium oxide | [% by mass] | 5.0 | 5.0 | 5.0 |
| | Total | | [% by mass] | 100.0 | 100.0 | 100.0 |
| | Carbonate + organic acid | | [% by mass] | 53.8 | 53.8 | 53.8 |
| | Carbonate + organic acid + surfactant | | [% by mass] | 77.3 | 77.3 | 77.3 |
| | [Organic acid/carbonate] | | mass ratio | 0.33 | 0.33 | 0.33 |
| | [(Carbonate + organic acid)/Surfactant] | | mass ratio | 2.3 | 2.3 | 2.3 |
| Preservation stability | | | [ml] | 50< | 1.9 | 4.5 |
| Texture | | | [-] | 1 | 5 | 5 |

*1 "Organic acid granulated" described in the section "Granulated state" in Tables 3 and 4, refers to a state in which the organic acid and all the components (surfactant, excipient, binder, and moisture absorbent) other than the carbonate (baking soda) were granulated. The resulting granulated particles correspond to the particles (B) containing the organic acid of the present invention. In the case of "Organic acid granulated", the carbonate (baking soda) was used as ungranulated starting material particles (which correspond to the particles (A) containing the carbonate).
   "Carbonate granulated" refers to a state in which the carbonate and all the components (surfactant, excipient, binder, and moisture absorbent) other than the organic acid (citric acid) were granulated. The resulting granulated particles correspond to the particles (A) containing the carbonate of the present invention. In the case of "Carbonate granulated", the organic acid (citric acid) was used as ungranulated starting material particles (which correspond to the particles (B) containing the organic acid).
   "Ungranulated" refers to a state in which all the powder components were used without being granulated.
*2 Carrageenan was dissolved in purified water and used as an aqueous solution of 1.6% carrageenan. However, the content of carrageenan in Tables 3 and 4 is indicated by a solid content because the purified water evaporates during the production process.
*3 The particle size in Tables 3 and 4 is indicated by a median diameter.

From Tables 3 and 4, the cleansing compositions of the present Examples are found to be excellent in preservation stability and excellent in texture.

All the cleansing compositions of the present Examples had favorable foamability, foam break, and long-lasting foam.

### Industrial Applicability

The present invention can provide a carbonic acid foaming composition and a powder or granular cleansing composition which have high preservation stability, exhibit reduced roughness in texture, and offer favorable feeling in use. The carbonic acid foaming composition can be used in combination with a hair cosmetic such as a shampoo or a conditioning agent so that the hair cosmetic is in a dosage form of foam. The cleansing composition can be used in a face care product such as a face wash, or a body care product such as a hand soap or a body soap.

## Claims

1. A carbonic acid foaming composition comprising a carbonate and an organic acid, wherein
the carbonic acid foaming composition comprises particles (A) comprising the carbonate and particles (B) comprising the organic acid,
the particles (A) are particles containing no organic acid, and the particles (B) are particles containing no carbonate,
a median diameter of carbonate particles (a) as a starting material of the particles (A) is 170 µm or smaller, and a median diameter of organic acid particles (b)as a starting material of the particles (B) is 500 µm or smaller,
a median diameter of particles of at least either the particles (A) or the particles (B) is 80 µm or larger,
the median diameter of the particles (A) is equal to or larger than the median diameter of the carbonate particles (a), and the median diameter of the particles (B) is equal to or larger than the median diameter of the organic acid particles (b).

2. The carbonic acid foaming composition according to claim 1, wherein the particles of at least either the particles (A) or the particles (B) comprise granulated particles obtained by granulating starting material particles.

3. The carbonic acid foaming composition according to claim 1 or 2, further comprising a moisture absorbent.

4. The carbonic acid foaming composition according to any one of claims 1 to 3, wherein the organic acid comprises one or more members selected from the group consisting of citric acid, succinic acid, tartaric acid, and ascorbic acid.

5. The carbonic acid foaming composition according to any one of claims 1 to 4, wherein the carbonate comprises one or more members selected from the group consisting of sodium carbonate and sodium bicarbonate.

6. The carbonic acid foaming composition according to any one of claims 3 to 5, wherein the moisture absorbent is magnesium oxide.

7. The carbonic acid foaming composition according to any one of claims 3 to 6, wherein the particles (A) or the particles (B) are granulated particles further comprising the moisture absorbent.

8. The carbonic acid foaming composition according to any one of claims 1 to 7, further comprising an excipient, wherein
the particles (A), the particles (B), or both the particles (A) and the particles (B) comprise the excipient.

9. The carbonic acid foaming composition according to any one of claims 1 to 8, wherein the median diameter of the particles (a) is 10 µm or larger and 150 µm or smaller, and the median diameter of the particles (b) is 10 µm or larger and 350 µm or smaller.

10. The carbonic acid foaming composition according to any one of claims 1 to 9, wherein the median diameter of the particles of at least either the particles (A) or the particles (B) is 90 µm or larger.

11. The carbonic acid foaming composition according to any one of claims 2 to 10, wherein a median diameter of the granulated particles is 110 µm or larger and 350 µm or smaller.

12. The carbonic acid foaming composition according to any one of claims 1 to 10, wherein a content of the carbonate in the carbonic acid foaming composition is 15% by mass or more and 65% by mass or less.

13. The carbonic acid foaming composition according to any one of claims 1 to 10, wherein a content of the organic acid in the carbonic acid foaming composition is 5% by mass or more and 60% by mass or less.

14. The carbonic acid foaming composition according to any one of claims 1 to 10, wherein a mass ratio of the organic acid to the carbonate [organic acid/carbonate] is 0.05 or more and 10 or less.

15. The carbonic acid foaming composition according to any one of claims 1 to 10, wherein a total amount of the carbonate and the organic acid in the carbonic acid foaming composition is from 50 to 90% by mass.

16. The carbonic acid foaming composition according to any one of claims 3 to 10, wherein a content of the moisture absorbent in the carbonic acid foaming composition is from 0.01 to 15% by mass.

17. The carbonic acid foaming composition according to any one of claims 1 to 10, wherein a value of a coefficient of variation, CV, represented by the following expression (1) in the particles (a) and the particles (b) is 95% or less:
Value of a coefficient of variation, CV (%) = [Standard deviation σ of a particle size] / [Median diameter D] × 100 (Expression 1).

18. The carbonic acid foaming composition according to any one of claims 1 to 10, wherein a content of 10 µm or smaller particles is 0.001% by mass or more and 3.5% by mass or less in all particles constituting the carbonic acid foaming composition.

19. A method for producing the carbonic acid foaming composition according to any one of claims 1 to 18, comprising a step of,
by using carbonate particles (a) having a median diameter of 170 µm or smaller and organic acid particles (b) having a median diameter of 500 µm or smaller as the starting materials, granulating at least either the carbonate particles (a) or the organic acid particles (b) to prepare granulated particles having a median diameter of 80 µm or larger.

20. A method for using the carbonic acid foaming composition according to any one of claims 1 to 18, comprising bringing the carbonic acid foaming composition into contact with a surfactant-containing composition to form the surfactant-containing composition into a dosage form of foam.

21. The method for using the carbonic acid foaming composition according to claim 20, wherein the surfactant-containing composition is a hair cosmetic.

22. A hair cosmetic kit comprising the carbonic acid foaming composition according to any one of claims 1 to 18 and a surfactant-containing composition.

23. A cleansing composition comprising a carbonate, an organic acid, and a surfactant, wherein
the cleansing composition comprises particles (A) comprising the carbonate and particles (B) comprising the organic acid,
the particles (A) are particles containing no organic acid, and the particles (B) are particles containing no carbonate,
a median diameter of carbonate particles (a) as a starting material of the particles (A), and a median diameter of organic acid particles (b) as a starting material of the particles (B) are 170 µm or smaller,
a median diameter of particles of at least either the particles (A) or the particles (B) is 80 µm or larger,
the median diameter of the particles (A) is equal to or larger than the median diameter of the carbonate particles (a), and the median diameter of the particles (B) is equal to or larger than the median diameter of the organic acid particles (b).

24. The cleansing composition according to claim 23, wherein the particles of at least either the particles (A) or the particles (B) comprise granulated particles obtained by granulating the starting material particles.

25. The cleansing composition according to claim 23 or 24, further comprising a moisture absorbent.

26. The cleansing composition according to any one of claims 23 to 25, wherein the organic acid comprises one or more members selected from the group consisting of citric acid, succinic acid, tartaric acid, and ascorbic acid.

27. The cleansing composition according to any one of claims 23 to 26, wherein the carbonate comprises one or more members selected from the group consisting of sodium carbonate and sodium bicarbonate.

28. The cleansing composition according to any one of claims 25 to 27, wherein the moisture absorbent is magnesium oxide.

29. The cleansing composition according to any one of claims 25 to 28, wherein the particles (A) or the particles (B) are granulated particles further comprising the surfactant and the moisture absorbent.

30. The cleansing composition according to claim 29, wherein the particles (A), the particles (B), or both the particles (A) and the particles (B) comprise the surfactant.

31. A method for producing the cleansing composition according to any one of claims 23 to 30, comprising a step of,
by using carbonate particles (a) having a median diameter of 170 µm or smaller and organic acid particles (b) having a median diameter of 170 µm or smaller as the starting materials, granulating at least either the carbonate particles (a) or the organic acid particles (b) to prepare granulated particles having a median diameter of 80 µm or larger.
